(19) Europäisches Patentamt
European Patent Office
Office européen des brevets

(11) EP 0 587 194 B1

(12) EUROPEAN PATENT SPECIFICATION

(45) Date of publication and mention of the grant of the patent:
**04.06.1997 Bulletin 1997/23**

(21) Application number: **93115719.2**

(22) Date of filing: **13.02.1990**

(51) Int Cl.[6]: **C07D 207/08**, C07D 207/14, C07D 207/26, C07D 207/28, C07D 211/22, C07D 211/42, C07D 211/46, C07D 211/70, C07D 213/30, C07D 217/04, C07D 233/54, C07D 241/04, C07D 259/00, C07D 265/30, C07D 279/12, C07D 295/08, C07D 453/02, A61K 31/445, A61K 31/415

(54) **2-(Heterocycloalkoxy)-1-(subst.-phenyl)-ethanol derivatives as cerebral function improving agents**

2-(Heterocycloalkoxy)-1-(Subst.-Phenyl)-Ethanol-Derivate als Wirkstoffe zur Verbesserung der cerebralen Funktion

Dérivés de 2-(hétérocycloalkoxy)-1-(phényl-subst.)-éthanol comme agents améliorant la fonction cérébrale

(84) Designated Contracting States:
**AT CH DE DK GB LI NL SE**

(30) Priority: **14.02.1989 JP 32714/89**
**20.03.1989 JP 68958/89**
**26.04.1989 JP 106187/89**
**05.02.1990 JP 24501/90**
**05.02.1990 JP 24502/90**
**05.02.1990 JP 24503/90**

(43) Date of publication of application:
**16.03.1994 Bulletin 1994/11**

(62) Application number of earlier application in accordance with Art. 76 EPC: **90102829.0**

(73) Proprietor: **TOYAMA CHEMICAL CO., LTD.**
**Tokyo 160 (JP)**

(72) Inventors:
- **Ono, Satoshi**
  **Toyama-shi (JP)**
- **Yamafuji, Tetsuo**
  **Nei-gun, Toyama-ken (JP)**
- **Chaki, Hisaaki**
  **Kaminiikawa-gun, Toyama-ken (JP)**
- **Maekawa, Mutsuko**
  **Toyama-shi (JP)**
- **Todo, Yozo**
  **Toyama-ken (JP)**
- **Narita, Hirokazu**
  **Toyama-shi (JP)**

(74) Representative:
**Wächtershäuser, Günter, Prof. Dr.**
**Patentanwalt,**
**Tal 29**
**80331 München (DE)**

(56) References cited:
**EP-A- 0 256 890** **DE-A- 2 058 206**

- **DATABASE WPI, Week 8629, Derwent Publications Ltd., London, GB; AN 86-186002; & JP-A-61 118 304**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

This invention relates to a 1,2-ethanediol derivative and a salt thereof, a process for producing the same, and a cerebral function-improving agent comprising the same. The cerebral function-improving agent of this invention is useful for treating cerebrovascular dementia, senile dementia, Alzheimer's dementia, sequelae of ischemic encephalopathy and cerebral apoplexy.

As 1,2-ethanediol derivatives, there are known, for example, those described in U.S. Patent No. 2,928,845; J. Pharm. Sci., vol. 50, pp. 769-771 (1961); Farmaco. Ed. Sci., vol. 19, pp. 1056-1065 (1964); etc.

These compounds are in use as a local anesthetic. However, nothing is known as to their use as a cerebral function-improving agent, an antiamnesic agent or a nootropic agent.

WO No. 88/8424 describes that 1,2-ethanediol derivatives can be used for the remedy of Alzheimer's disease and other degenerative neurological disorders. However, no specific description or example of these derivatives is given in the application.

JP-A-61118304 discloses 1,2-ethanediol derivatives wherein a 1,2-ethanediol group is linked directly to one of the carbon atoms of a pyrazole ring.

DE-A-30 03 323, US-A-4,237,138 and Campbell et al., J. Med. Chem., Vol. 31 (1988), pp. 516-520 disclose piperidine derivatives, wherein a 1,2-ethanediol moiety is linked directly to one of the carbon atoms of the piperidine ring.

Vodicka et al., Carcinogenesis, Vol. 9, No. 9 (1988), pp. 1657-1660 and Moschel et al., J. Org. Chem., Vol. 51 (1986), pp. 2952-2955 disclose $O^6$-(2-hydroxy-2-phenylethyl)deoxyguanosine.

Winterfeld et al., Archiv der Pharmazie, Vol. 296 (1963), pp. 38-47 discloses 1-phenyl-2-(piperidin-2-methyloxy) ethanol.

Baker, J. Org. Chem., Vol. 50 (1985), pp. 3942-3943 discloses 1-phenyl-2-(pyridin-2-yloxy)ethanol.

Baiocchi et al., Rend. Accad. Nat. 40 (Quaranta) [4] 18-19 (1968), pp. 269-280 discloses 1-phenyl-2-(1-benzyl-1H-indazol-3-yloxy)-ethanol.

Drugs such as cerebral metabolic enhancers, cerebrovasodilators and the like are currently in use for the remedy of various dementias, particularly dementia of Alzheimer's type and cerebrovascular dementia.

However, no cerebral function-improving agent has been found as yet which is useful for treating cerebrovascular dementia, senile dementia, Alzheimer's dementia, sequelae of ischemic encephalopathy and cerebral apoplexy.

An object of this invention is to provide a novel 1,2-ethanediol derivative and a salt thereof.

Another object of this invention is to provide a process for producing a novel 1,2-ethanediol derivative and a salt thereof.

Still another object of this invention is to provide a novel cerebral function-improving agent which is useful for treating cerebrovascular dementia, senile dementia, Alzheimer's dementia, sequelae of ischemic encephalopathy and cerebral apoplexy and yet has little side effect.

The present inventors have made study in order to solve the above-mentioned problems. As a result, it has been found that a 1,2-ethanediol derivative represented by the following general formula [I] or a salt thereof has an excellent antiamnesic activity and an excellent antihypoxic activity and is very useful as a cerebral function-improving agent:

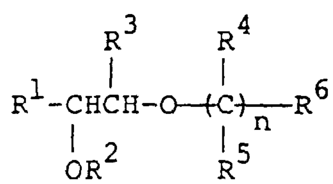

[I]

wherein $R^1$ represents a substituted or unsubstituted phenyl group; $R^2$ represents a hydrogen atom, a $C_{1-6}$ alkyl group or a hydroxyl-protecting group; $R^3$ represents a hydrogen atom or a $C_{1-6}$ alkyl group; $nR^4$'s and $nR^5$'s are the same as or different from one another and represent hydrogen atoms or $C_{1-6}$ alkyl groups; n represents an integer of 1 to 6; and $R^6$ represents a substituted or unsubstituted nitrogen-containing heterocyclic group, the nitrogen-containing heterocyclic group attaching to carbon atom through a carbon atom forming the heterocyclic ring and being selected when n represents 1 from the group consisting of pyrrolyl, pyrrolidinyl, piperazinyl, imidazolyl, pyridyl, tetrahydropyridyl, pyrimidinyl, morpholinyl, thiomorpholinyl, quinolyl, quinolizinyl, tetrahydroquinolinyl, tetrahydroisoquinolinyl, quinuclidinyl, thiazolyl, tetrazolyl, thiadiazolyl, pyrrolinyl, imidazolinyl, imidazolidinyl, pyrazolinyl, pyrazolidinyl, purinyl and indazolyl groups or being selected when n represents an integer of 2 to 6 from the group consisting of pyrrolyl, pyrrolidinyl, piperidyl, piperazinyl, imidazolyl, pyridyl, tetrahydropyridyl, pyrimidinyl, morpholinyl, thiomorpholinyl, quinolyl, quinolizinyl, tetrahydroquinolinyl, tetrahydroisoquinolinyl, quinuclidinyl, thiazolyl, tetrazolyl, thiadiazolyl, pyrrolinyl, imidazolinyl, imidazolidinyl, pyrazolinyl, pyrazolidinyl, purinyl and indazolyl groups, wherein the substituent on $R^1$ is selected from the group consisting of halogen atoms, substituted or unsubstituted amino, $C_{1-6}$ alkyl, aryl, ar-$C_{1-4}$ alkyl, $C_{1-6}$ alkoxy, ar-$C_{1-4}$ alkoxy, aryloxy, carbamoyloxy, $C_{1-6}$ alkylthio, $C_{2-6}$ alkenyl, $C_{2-6}$ alkenyloxy, ar-$C_{1-4}$ alkylthio, ar-$C_{1-6}$ alkylsulfonyl, arylsulfonyl, $C_{1-6}$ alkylsulfonylamino, arylsulfonylamino and heterocyclic groups, protected amino groups, protected or unprotected hydroxyl groups, nitro group, oxo group and $C_{1-4}$ alkylenedioxy groups; the substituted $C_{1-6}$ alkyl, aryl, ar-$C_{1-4}$ alkyl, $C_{1-6}$ alkoxy, ar-$C_{1-4}$ alkoxy, aryloxy, carbamoyloxy, $C_{1-6}$ alkylthio, $C_{2-6}$ alkenyl, $C_{2-6}$ alkenyloxy, ar-$C_{1-4}$ alkylthio, ar-$C_{1-6}$ alkylsulfonyl, arylsulfonyl, $C_{1-6}$ alkylsulfonylamino, arylsulfonylamino or heterocyclic group as the substituent of $R^1$ and the substituted nitrogen-containing heterocyclic group as $R^6$ have each at least one substituent selected from the group consisting of halogen atoms, protected or unprotected hydroxyl groups, protected or unprotected amino groups, protected or unprotected carboxyl groups, unsubstituted $C_{1-6}$ alkyl groups, $C_{1-6}$ alkyl groups substituted by a protected or unprotected hydroxyl group, unsubstituted or halogen-substituted aryl groups, unsubstituted or halogen-substituted aroyl groups, unsubstituted $C_{1-6}$ alkoxy groups, $C_{1-6}$ alkoxy groups substituted by a $C_{1-6}$ alkoxy group, $C_{1-6}$ acyl groups, ar-$C_{1-4}$ alkyl groups, ar-$C_{2-6}$ alkenyl groups, heterocyclic groups, heterocyclic-CO-groups, oxo group, $C_{1-6}$ alkylsulfonyl groups and arylsulfonyl groups; and the substituted amino group as the substituent of $R^1$ has each at least one substituent selected from the group consisting of protected or unprotected hydroxyl groups, unsubstituted $C_{1-6}$ alkyl groups, $C_{1-6}$ alkyl groups substituted by a protected or unprotected carboxyl or hydroxyl group, cycloalkyl groups, aryl groups, $C_{1-6}$ acyl groups, ar-$C_{1-4}$ alkyl groups, heterocyclic groups, unsubstituted or oxo-substituted heterocyclic-CO- groups, adamantyl group, $C_{1-6}$ alkylsulfonyl groups and arylsulfonyl groups; all the above heterocyclic groups being selected from the group consisting of the nitrogen-containing heterocyclic groups mentioned in the definition of $R^6$ and furyl, thienyl, benzothienyl, pyranyl, isobenzofuranyl, oxazolyl, benzofuranyl, indolyl, benzimidazolyl, benzoxazolyl, benzothiazolyl, quinoxalyl, dihydroquinoxalyl, 2,3-dihydrobenzothienyl, 2,3-dihydrobenzopyrrolyl, 2,3-dihydro-4H-1-thianaphthyl, 2,3-dihydrobenzofuranyl, benzo[b]dioxanyl, imidazo[2,3-a]pyridyl, benzo[b]piperazinyl, chromenyl, isothiazolyl, isoxazolyl, oxadiazolyl, pyridazinyl, isoindolyl and isoquinolyl groups.

Incidentally, the cerebral-function-improving agent mentioned herein refers to a cerebral-function-improving agent having not only effects possessed by conventional cerebral-function-improving agents, for example, sequelae of ischemic encephalopathy and cerebral apoplexy but also therapeutic or prophylactic effects for amnesia and dementias (e.g. cerebrovascular dementia, senile dementia and Alzheimer's dementia).

In the present specification, the following terms have the following definitions unless otherwise specified.

The term "halogen atom" means, for example, a fluorine atom, a chlorine atom, a bromine atom and an iodine atom; the term "lower alkyl group" means $C_{1-6}$ alkyl groups such as methyl, ethyl, n-propyl, isopropyl, n-butyl, isobutyl, tert-butyl, pentyl, hexyl and the like; the term "lower alkoxy group" means $C_{1-6}$ alkyl-O- groups; the term "lower alkylthio group" means $C_{1-6}$ alkyl-S-groups; the term "lower alkenyl group" means $C_{2-6}$alkenyl groups such as vinyl, propenyl, butenyl, pentenyl, hexenyl and the like; the term "lower alkenyloxy group" means $C_{2-6}$alkenyl-O- groups; the term "cycloalkyl group" means $C_{3-6}$cycloalkyl groups such as cyclopropyl, cyclobutyl, cyclopentyl, cyclohexyl and the like; the term "aryl group" means phenyl, naphthyl, indanyl and indenyl groups; the term "aryloxy group" means aryl-O-groups; the term "ar-lower alkyl group" means ar-$C_{1-4}$ alkyl groups such as benzyl, diphenylmethyl, trityl, phenethyl and the like; the term "ar-lower alkoxy group" means ar-$C_{1-4}$alkyl-O- groups; the term "ar-lower alkylthio group" means aryl-$C_{1-4}$alkyl-S- groups; the term "lower alkylenedioxy group" means $C_{1-4}$alkylenedioxy groups such as methylenedioxy, ethylenedioxy and the like; the term "lower acyl group" means $C_{1-6}$acyl groups such as formyl, acetyl, butyryl and the like; the term "aroyl group" means aryl-CO- groups; the term "lower alkylsulfonyl group" means $C_{1-6}$alkyl-$SO_2$-groups; the term "arylsulfonyl group" means aryl-$SO_2$- groups; ther term "ar-lower alkylsulfonyl group" means aryl-$C_{1-6}$alkyl-$SO_2$- groups; the term "lower alkylsulfonyloxy group" means $C_{1-6}$alkyl-$SO_2$-O- groups; the term "aryl-sulfonyloxy group" means aryl-$SO_2$-O- groups; the term "lower alkylsulfonylamino group" means $C_{1-6}$alkyl-$SO_2$-NH-groups; the term "arylsulfonylamino group" means aryl-$SO_2$NH- groups; the term "di-lower alkylamino group" means di-$C_{1-6}$alkyl-NH- groups and the term "ammonio group" means tri-lower alkylammonio groups such as trimethylammonio, triethylammonio; the term "lower alkoxycarbonyl group" means $C_{1-6}$alkyl-O-CO- groups and the like.

The protective groups or hydroxyl group, carboxyl group and amino group include those conventional protective groups for hydroxyl group, carboxyl group and amino group which are described in Protective Groups in Organic Synthesis [Theodra W. Greene (1981), John Wiley & Sons, Inc.]. In particular, the protective group for hydroxyl group specifically includes, for example, lower alkyl, lower acyl, tetrahydropyranyl and ar-lower alkyl groups such as substituted or unsubstituted benzyl.

The salt of the 1,2-ethanediol derivative represented by the general formula [I] can be any pharmaceutically acceptable salt. It includes, for example, salts with mineral acids such as hydrochloric acid, hydrobromic acid, sulfuric acid, phosphoric acid and the like; salts with carboxylic acids such as formic acid, acetic acid, oxalic acid, fumaric acid, maleic acid, malic acid, tartaric acid, aspartic acid and the like; salts with sulfonic acids such as methanesulfonic acid, benzenesulfonic acid, p-toluenesulfonic acid, naphthalene-sulfonic acid and the like; and salts with alkali metals such as sodium, potassium and the like.

When the 1,2-ethanediol derivative of the general formula [I] or its salt has isomers (e.g. optical isomers, geometrical isomers, tautomers) , all of these isomers are included in this invention. Also, the hydrate, solvate and all crystal forms of the compound of this invention are included in this invention.

Next, there is described a process for producing a 1,2-ethanediol derivative of the general formula [I] or a salt thereof.

The 1,2-ethanediol derivative of the general formula [I] or its salt can be produced by per se known processes or their appropriate combinations, for example, the following production processes.

Production Process 1

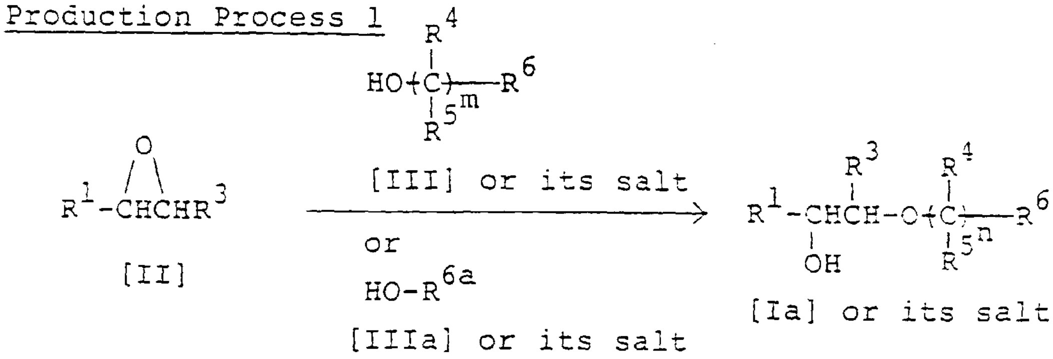

[II]

[III] or its salt

or

[IIIa] or its salt

[Ia] or its salt

In the above reaction schemes, $R^1$, $R^3$, $R^4$, $R^5$, $R^6$ and n have the same meanings as defined above; and m represents an integer of 1-6.

As the salts of the compounds of the general formulas [III] [IIIa] and [Ia],
there can be mentioned the same salts as the salts of the compound of the general formula [I].

Next, each of the above production processes is described.

Production Process 1

A compound of the general formula [II] is reacted with a compound of the general formula [III] or its salt or a compound of the general formula [IIIa] or its salt in the presence or absence of a base to obtain a compound of the general formula [Ia] or its salt.

The solvent to be used in this reaction can be any solvent unless it adversely affects the reaction. There can be mentioned, for example, aromatic hydrocarbons such as benzene, toluene, xylene and the like; sulfoxides such as dimethylsulfoxide and the like; amides such as N,N-dimethylformamide and the like; and ethers such as tetrahydrofuran, dioxane and the like. These solvents can be used alone or in admixture of two or more. It is also possible to use the compound of the general formula [III] cr [IIIa] as the solvent.

As the base to be used optionally, there can be mentioned, for example, sodium hydride, metallic sodium and potassium tert-butoxide.

In the reaction, the compound of the general formula [III] or its salt, or the compound of the general formula [IIIa] or its salt is used in an amount of 1-100 moles, preferably 1-10 moles, per mole of the compound of the general formula [II].

The base as an optional component is used in an amount of 0.01-1.2 moles per mole of the compound of the general formula [II].

This reaction can be effected usually at 20-150°C, preferably 70-90°C, for 1 minutes to 24 hours, preferably 5 minutes to 5 hours.

In the above production process, when the compounds of the general formulas [II], [III] and [IIIa], have isomers (e.g. optical isomers, geometrical isomers, tautomers), the compounds can be used in any isomer form. Further, the compounds can be used in a hydrate form, a solvate or or any crystal form.

When the compounds of the general formulas [II], [III], [IIIa], [I] and [Ia] have a hydroxyl group, an amino group or a carboxyl group, it is possible to previously protect these groups with a conventional protective group and to remove, after the reaction, the protective group, if necessary, according to a per se known method.

The compound thus obtained may be subjected to conventional isolation and purification procedures such as column chromatography, crystallization, distillation, extraction and the like.

The 1,2-ethanediol derivative of the general formula [I] or its salt can be converted into other 1,2-ethanediol derivative of the general formula [I] or its salt by subjecting the former compound to appropriate combination of per se known reactions such as oxidation reaction, reduction reaction, addition reaction, acylation reaction, alkylation reaction, sulfonylation reaction, deacylation reaction, substitution reaction, dehydration reaction, hydrolysis reaction and the like.

The compound of the general formula [II] which is the starting material for producing the compound of this invention can be produced by per se known processes, for example, the process described in JACS, vol. 87, p 1353 (1965) and the process described in Shin Jikken Kagaku Koza, vol. 14, p. 579 (1977), Maruzen.

The compound of this invention, when used as a drug, may be appropriately mixed with excipients such as filler, carrier, diluent and the like and can be formed into tablets, capsules, powders, granules, fine granules, pills, suspensions, emulsions, liquids, syrups, injections, etc. according to conventional methods. These drugs can be administered orally or parenterally. The dosage route, dose and number of administrations can be appropriately varied depending upon the age, weight and symptom of patient, but in the case of oral administration, generally 0.01-500 mg of the present compound can be administered daily to an adult patient in one to several portions.

Next, the pharmacological activities of representative compounds of this invention are described.

The numbers of the test compounds used in the following pharmacological tests refer to the numbers of the compounds shown in Production Examples appearing hereinafter.

1. Effect of test compound on hypoxia

A test compound dissolved in physiological saline (100 mg/kg) was orally administered to a ddY female mouse (5-6 weeks old, each group consisting of 10 mice). After 1 (or 30 minutes*) hour from the administration, each mouse was placed in a 300-ml glass chamber, and a 5 gas mixture consisting of 4% of oxygen and 96% of nitrogen was passed through the chamber at a rate of 5 liters/min. A time from the start of gas passing to the death of each mouse was measured.

To a control mice group was orally administered only physiological saline.

The antihypoxic activity of the test compound was calculated from the following formula:

$$\frac{\text{Mean survival time of mice of administration group}}{\text{Mean survival time of mice of control group}} \times 100\ (\%)$$

The results are shown in Table 1.

Table 1

| Compound No. | Antihypoxic activity (%) | Compound No. | Antihypoxic activity (%) |
|---|---|---|---|
| 61 | 184* | 134 | 201* |
| 62 | 127 | 137 | 163* |
| 119 | 175* | 139 | 162* |
| 120 | 162* | 151 | 182 |
| 121 | 149* | 152 | 180 |
| 122 | 142* | 153 | 185 |
| 123 | 140* | 215 | 197 |
| 125 | 172* | 218 | 157* |
| 126 | 172* | 219 | 222* |
| 129 | 158* | 220 | 183* |
| 131 | 167* | 221 | 183* |
| 132 | 133* | | |

Note: * Mice were placed in the chamber after 30 minutes from the administration instead of after 1 hr from the administration.

2. Effect of test compound on amnesia

(1) Electroconvulsive shock (ECS)-induced amnesia

A test compound dissolved in physiological saline was intraperitoneally administered to a ddY male mouse (5-6 weeks old, each group consisting of 10 mice). The acquisition trial in passive avoidance task was carried out after 1 hour from the administration. Each mouse was placed in the bright compartment of a two-compartment step-through type passive avoidance apparatus consisting of a bright compartment and a dark compartment (MPA-100M manufactured by Muromachi Kikai). When the mouse entered the dark compartment, the guilotine door of the dark compartment was closed; after 0.5 second, an inescapable footshock (1.6 mA, 3 seconds) was delivered. Immediately thereafter, ECS (25 mA, 0.5 second) was applied through the both eyes of the mouse. After 24 hours, in the retention trial, the mouse was again placed in the bright compartment and the response latency for mouse to enter the dark compartment was measured. If the mouse avoided longer than 300 seconds, a ceiling score of 300 seconds was assigned.

A control mice group to which only physiological saline had been administered intraperitoneally, and response latency was also measured in the same manner.

Antiamnesic activity was taken as a median of the response latencies of the 10 mice and expressed by the following symbols:

- : 0-60 seconds

+ : 61-100 seconds

++ : 101-150 seconds

+++ : 151-300 seconds

The results are shown in Table 2.

Table 2

| Compound No. | Dosage (mg/Kg) | Antiamnesic activity |
|---|---|---|
| 61 | 3 | ++ |

(2) Effect of test compound on cycloheximide-induced amnesia

It was reported by Yamazaki et al. [Drugs, Mind and Action, vol. 3, pp. 127-136 (1983)] that cycloheximide interfers with the retrieval process of memory of mouse. Hence, the following test was carried out.

A test was carried out in accordance with the method described in Drugs, Mind and Action, vol. 3, pp. 127-136 (1983) and Folia Pharmacologica Japonica, vol. 89, pp. 243-252 (1987).

As the test apparatus, there was used a step-down type passive avoidance training box. It was a black acrylic resin box of 22 cm x 22 cm x 21 cm (height) whose floor portion consisted of a stainless steel grid and which had, at one corner of the floor grid, a platform of 7 cm x 7 cm x 2 cm (height).

Cycloheximide was dissolved in physiological saline, and injected subcutaneously at 120 mg/kg to a ddY male mouse (5-6 weeks old, each group consisting of 10 mice). In an acquisition trial, after 15 minutes from the administration, each mouse was placed on the platform in the above test apparatus. As soon as the mouse stepped down the platform, a 2 mA current was delivered for 2 seconds, immediately after which the mouse was returned to its home cage. A retention test was performed 24 hours after the acquisition. To each mouse which had been treated with cycloheximide was orally administered a test compound dissolved in physiological saline; after 30 minutes from the administration, the mouse was again placed on the platform and the response latency for mouse to step down was measured. If the mouse avoided longer than 300 seconds, a ceiling score of 300 seconds was assigned.

A control mice group to which only physiological saline had been administered orally, was also subjected to the measurement of response latency in the same manner.

Antiamnesic activity was taken as a median of the response latencies of the 10 mice and expressed by the following symbols:

- : 0-60 seconds
+ : 61-100 seconds
++ : 101-150 seconds
+++ : 151-300 seconds

The results are shown in Table 3.

Table 3

| Compound No. | Dosage (mg/Kg) | Antiamnesic activity |
|---|---|---|
| 60 | 3 | +++ |

Table 3 (continued)

| Compound No. | Dosage (mg/Kg) | Antiamnesic activity |
|---|---|---|
| 147 | 3 | +++ |
| 151 | 3 | +++ |
| 153 | 3 | ++ |
| 219 | 3 | + |
| 220 | 10 | ++ |
| 221 | 10 | + |
| Control | - | - |

3. Inhibitory activity for acetylcholinesterase

A test was conducted in accordance with the method of Ellman et al. [Biochem. Pharmacol., vol. 7, pp. 88-95, 1961].

That is, acetylthiocholine (as a substrate) was added to a phosphate buffer solution containing 5,5'-dithiobis-(2-nitrobenzoic acid) (DTNB), a test compound and a mouse cerebral homogenate (as an acetylcholinesterase source). The resulting mixture was incubated, and the amount of the resulting 5-thio-2-nitrobenzoic acid was photometrically measured at 412 nm.

The inhibitory activity for acetylcholinesterase of the test compound was expressed by the inhibition (%) when the final concentration of the test compound was 10 μg/ml.

The results are shown in Table 4.

Table 4

| Compound No. | Inhibition (%) | Compound No. | Inhibition (%) |
|---|---|---|---|
| 60 | 71 | 78 | 90 |
| 61 | 89 | 341 | 61 |
| 62 | 78 | 342 | 67 |

4. Acute toxicity

A test compound dissolved in physiological saline was intravenously administered to a group of 3 ddY male mice (5-6 weeks old) to examine the acute toxicity of the test compound.

As a result, test compound Nos. 119, 120, 126, 132, 147, 151, 176, 219, 220 and 221, gave no death case at 50 mg/kg.

It is easily appreciated from the above test results that the compound of this invention is excellent in antihypoxic activity, antiamnesic activity and inhibitory activity for acetylcholinesterase and has low toxicity.

From the above result, it is also easily appreciated that the cerebral function-improving agent of this invention is useful for treating cerebrovascular dementia, senile dementia, Alzheimer's dementia, sequelae of ischemic encephalopathy and cerebral apoplexy.

Next, the process for producing the compound of this invention is specifically described by way of Production Examples.

In the Production Examples, the mixing ratio of eluant is by volume in all cases and, as the carrier in column chromatography, there was used a silica gel (Kieselgel 60, Art. 7734) manufactured by Merck Co.

The abbreviations used in the Production Examples have the following meanings.

Me: methyl, Et: ethyl, i-Pr: isopropyl, t-Bu: tert-butyl, Ac: acetyl, Ph: phenyl, DPM: diphenylmethyl, Bz: benzyl, Tr: trityl, IPA: isopropyl alcohol, IPE: diisopropyl ether, PTS: p-toluenesulfonic acid.

In the following sentences and tables, the substances in [ ] refer to solvents used in recrystallization.

Production example 1

(1) A mixture of 10.8 g of (L)-benzoylcystine, 1.6 g of lithium borohydride, 2.4 g of tert-butanol and 180 ml of tetrahydrofuran was refluxed for 1 hour and then cooled to -60°C. Thereto was added 6.1 g of 4-benzyloxyphenacyl bromide. The mixture was stirred for 30 minutes at the same temperature and further for 3 hours at -40° to -30°C. The reaction mixture was added to a mixture of 100 ml of water and 200 ml of diethyl ether. The organic layer was separated, washed with water, a saturated aqueous sodium hydrogencarbonate solution and a saturated aqueous sodium chloride solution in this order, and dried over anhydrous magnesium sulfate. The solvent was removed by

distillation under reduced pressure. The residue thus obtained was purified by column chromatography (eluant: toluene) to obtain 2.8 g of (S)-1-(4-benzyloxyphenyl)-2-bromoethanol.

(2) 2.8 g of (S)-1-(4-benzyloxyphenyl)-2-bromoethanol was dissolved in a mixed solvent of 20 ml of methanol and 10 ml of tetrahydrofuran. To the solution was added a solution of 0.8 g of potassium hydroxide dissolved in 4 ml of water, with ice cooling. The mixture was stirred for 5 minutes at the same temperature and further for 10 minutes at room temperature. The reaction mixture was added to a mixture of 50 ml of diethyl ether and 50 ml of ice water. The organic layer was separated. The aqueous layer was extracted with 25 ml of diethyl ether. The extract was combined with the previously separated organic layer. The combined organic layer was washed with water and a saturated aqueous sodium chloride solution in this order, and dried over anhydrous magnesium sulfate. The solvent was removed by distillation under reduced pressure to obtain 1.4 g of (S)-2-(4-benzyloxyphenyl)oxirane.

Melting point: 56-61°C
Optical rotation: $[\alpha]_D^{25}$ = +5.2° (C=2, $CHCl_3$)

The following compounds were obtained in the same manner.

o(S)-2-(3-Methylphenyl)oxirane
$[\alpha]_D^{25}$ = +15.7° (C=2, $CHCl_3$)
o(S)-2-(4-Phenoxyphenyl)oxirane
$[\alpha]_D^{25}$ = +12.5° (C=4, $CHCl_3$)

Production Example 2

(1) A solution of 23 g of (+)-diisopinocamphenylchloroborane dissolved in 30 ml of tetrahydrofuran was cooled to -25°C. Thereto was added 12 g of 4-benzyloxyphenacyl bromide. The resulting mixture was stirred for 4 hours at -20° to -15°C. The reaction mixture was added to a mixture of 150 ml of diethyl ether and 100 ml of ice water. The organic layer was separated, washed with water and a saturated aqueous sodium chloride solution in this order, and dried over anhydrous magnesium sulfate. The solvent was removed by distillation under reduced pressure. The residue thus obtained was purified by column chromatography (eluant: hexane:toluene = 1:2) to obtain 6.8 g of (R)-1-(4-benzyloxyphenyl)-2-bromoethanol.

(2) 6.0 g of (R)-1-(4-benzyloxyphenyl)-2-bromoethanol was dissolved in a mixed solvent of 50 ml of methanol and 25 ml of tetrahydrofuran. Thereto was added a solution of 1.5 g of potassium hydroxide dissolved in 5 ml of water, with ice cooling. The resulting mixture was stirred for 5 minutes at the same temperature and further for 10 minutes at room temperature. The reaction mixture was added to a mixture of 100 ml of diethyl ether and 100 ml of ice water. The organic layer was separated. The aqueous layer was extracted with 50 ml of diethyl ether. The extract was combined with the previously separated organic layer. The combined organic layer was washed with water and a saturated aqueous sodium chloride solution in this order, and dried over anhydrous magnesium sulfate. The solvent was removed by distillation under reduced pressure to obtain 3.7 g of (R)-2-(4-benzyloxyphenyl)oxirane.

Melting point: 58-67°C
Optical rotation: $[\alpha]_D^{26}$ = -14.4° (C=2, $CHCl_3$)

The following compounds were obtained in the same manner.

o(R)-2-(3-Methylphenyl)oxirane
$[\alpha]_D^{27}$ = -18.6° (C=2, $CHCl_3$)
o(R)-2-(4-Phenoxyphenyl)oxirane
$[\alpha]_D^{25}$ = -11.3° (C=2, $CHCl_3$)

Production Example 3 (Reference)

3.4 g of potassium tert-butoxide was added to 31 ml of 2-(N,N-dimethylamino)ethanol. The resulting mixture was heated to 80°C. Thereto was dropwise added 7.7 g of 2-(3-fluorophenyl)oxirane over 40 minutes. The mixture was stirred for 3 hours at the same temperature. The reaction mixture was added to a mixture of 200 ml of ice water and 200 ml of ethyl acetate. The organic layer was separated. To the organic layer was added 50 ml of water. The mixture was adjusted to pH 1 with 6 N hydrochloric acid. The aqueous layer was separated and mixed with 100 ml of chloroform. The resulting mixture was adjusted to pH 11 with a 2 N aqueous sodium hydroxide solution. The organic layer was separated, washed with water, and dried over anhydrous magnesium sulfate. The solvent was removed by distillation

under reduced pressure. The residue thus obtained was purified by column chromatography (eluant: chloroform/ethanol = 5/1). The resulting oily product was dissolved in 25 ml of acetone. Hydrogen chloride gas was blown into the solution. The resulting crystals were collected by filtration, washed with acetone and dried to obtain 3.1 g of 2-[2-(N,N-dimethylamino)ethoxy]-1-(3-fluorophenyl)ethanol hydrochloride

Melting point: 164-165°C [EtOH]

The compounds shown in Table 5 were obtained in the same manner.

In Table 5, $R^1$, $R^2$, $R^3$, $R^{4a}$, $R^{4b}$, $R^6$, na and nb each show a substituent or integer used in the following formula.

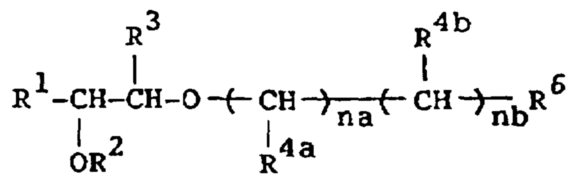

Table 5

| Compound No. | $R^1$ | $R^2$ | $R^3$ | $R^{4a}$ | $R^{4b}$ | $R^6$ | na | nb | Addition salt | Melting point (°C) |
|---|---|---|---|---|---|---|---|---|---|---|

Table 5

| | | | | | | | | | |
|---|---|---|---|---|---|---|---|---|---|
| 60 | 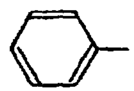 | H | H | H | H | 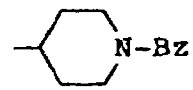 | 1 | 1 | - | Oily |
| 61 | 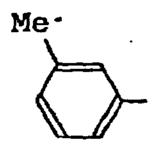 | " | " | " | " | " | " | " | " | " |
| 62 | 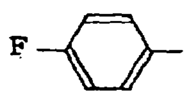 | " | " | " | " | " | " | " | " | 50-52 |

Table 5

| | | | | | | | | | | |
|---|---|---|---|---|---|---|---|---|---|---|
| 78 | 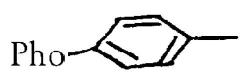 | " | " | " | " | 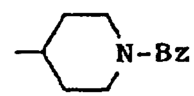 | " | " | - | Oily |

Production Example 4 (Reference)

A mixture of 5.00 g of 1-benzyl-4-hydroxypiperidine, 1.97 g of potassium tert-butoxide and 4 ml of dimethyl sulfoxide was heated to 80°C. Thereto was dropwise added 2.10 g of styrene oxide over 40 minutes. The resulting mixture was stirred for 3 hours at the same temperature. The reaction mixture was added to a mixture of 100 ml of ice water and 80 ml of ethyl acetate. The mixture was adjusted to pH 11.5 with 6 N hydrochloric acid. The organic layer was separated, washed with water and a saturated aqueous sodium chloride solution in this order, and dried over anhydrous magnesium sulfate. The solvent was removed by distillation under reduced pressure. The residue thus obtained was purified by column chromatography (eluant: chloroform/ethanol = 10/1). The resulting oily product was dissolved in 8 ml of ethanol. To the solution were added 1 ml of a 6 N dry hydrogen chloride-ethanol solution and 20 ml of diethyl ether. The mixture was stirred for 30 minutes at room temperature. The resulting crystals were collected by filtration, washed with a mixture of 2 ml of ethanol and 2 ml of diethyl ether, and dried to obtain 930 mg of 2-(l-benzylpiperidin-4-yloxy)-1-phenylethanol hydrochloride.

Melting point: 193-195°C

The compounds shown in Table 6 were obtained in the same manner.
In Table 6, $R^1$, $R^2$, $R^3$, $R^4$, $R^6$ and n each show a substituent or integer used in the following formula:

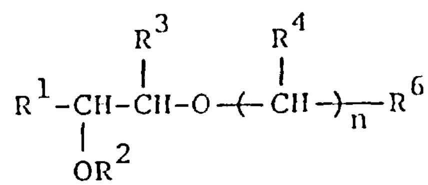

Table 6

| Compound No. | $R^1$ | $R^2$ | $R^3$ | $R^4$ | $R^6$ | n | Addition salt | Melting point (°C) |
|---|---|---|---|---|---|---|---|---|

Table 6 (cont'd)

| | | | | | | | | |
|---|---|---|---|---|---|---|---|---|
| 118 | 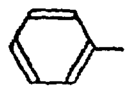 | H | H | H | 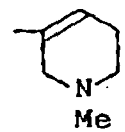 | 1 | HCl | Oily |
| 119 | " | " | " | " | 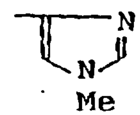 | " | - | 92-95 |
| 120 | " | " | " | " | 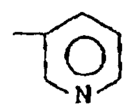 | " | HCl | Oily |

Table 6 (cont'd)

| | | | | | | | | |
|---|---|---|---|---|---|---|---|---|
| 121 | 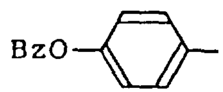 | H | H | H | 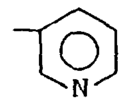 | 1 | - | 78-81 [AcOEt-IPE] |
| 122 | 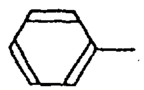 | " | " | " | 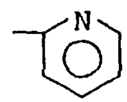 | " | HCl | 129.5-131.5 [AcOEt-IPE] |
| 123 | " | " | " | " | 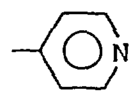 | " | " | 157.5-159.5 |

Table 6 (cont'd)

| | | | | | | | | |
|---|---|---|---|---|---|---|---|---|
| 125 | 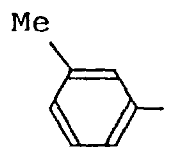 | H | H | H | 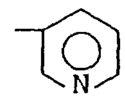 | 1 | HCl | Oily |
| 126 | 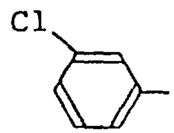 | " | " | " | " | " | " | " |
| 127 | 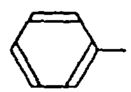 | " | " | " | 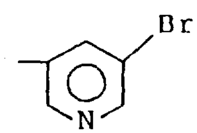 | " | " | " |
| 128 | " | " | " | " | 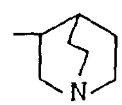 | " | – | " |

Table 6 (cont'd)

| | | | | | | | | |
|---|---|---|---|---|---|---|---|---|
| 129*1 | 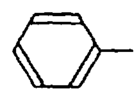 (S-form) | H | H | H | 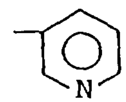 | 1 | – | 61-61.5 [EtOH-IPE] |
| 130*2 | " (R-form) | " | " | " | " | " | " | 61-61.5 [EtOH-IPE] |
| 131 | 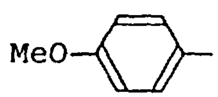 | " | " | " | " | " | " | 71.5-72 [EtOH-IPE] |
| 132*3 | 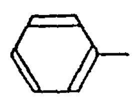 | " | " | " | 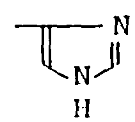 | " | " | 118-119.5 |

Table 6 (cont'd)

| | | | | | | | | |
|---|---|---|---|---|---|---|---|---|
| 134*3 | 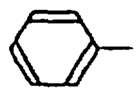 (1R-form) | H | H | H | 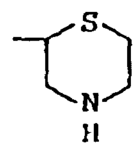 | 1' | HCl | Oily |
| 135*3 | 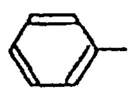 | " | " | " | 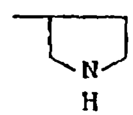 | " | " | 104.5-106.5 [IPA-AcOEt] |

Table 6 (cont'd)

| | | | | | | | | |
|---|---|---|---|---|---|---|---|---|
| 137*3 | 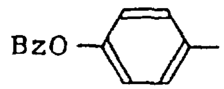 | H | H | H | 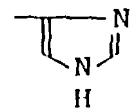 | 1 | - | 154-156 [IPA-AcOEt] |
| 138 | 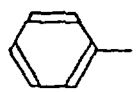 | " | " | " | 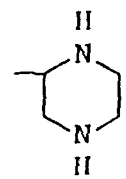 | " | 2HCl | 241-242 (decomp.) |
| 139 | 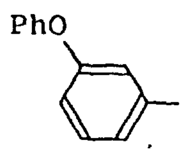 | " | " | " | 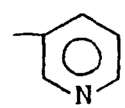 | " | - | 53.5-54.5 [$Et_2O$] |
| 140 | 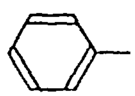 | " | " | " | " | 3 | " | Oily |

Table 6 (cont'd)

| | | | | | | | | |
|---|---|---|---|---|---|---|---|---|
| 141 | 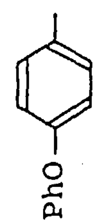 | H | H | H | 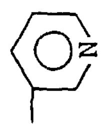 | 1 | - | 84.5-85.5 [EtOH-IPE] |
| 142 | 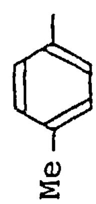 | " | " | " | " | " | " | 65.5-66.5 [EtOH-IPE] |
| 143 | 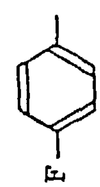 | " | " | " | " | " | " | 68-69 [EtOH-IPE] |

Note: *1: Optical rotation: +23.3° (25°C, C=1, MeOH)
*2: Optical rotation: -22.5° (25°C, C=1, MeOH)
*3: The reaction was effected using a trityl group as an amino-protecting group.
*4: The reaction was effected using a formyl group as an amino-protecting group.

Production Example 5

(1) A mixture of 4.30 g of 4-benzyl-2-hydroxymethyl-morpholine, 930 mg of potassium tert-butoxide and 4 ml of dimethyl sulfoxide was heated to 80°C. Thereto was dropwise added 2.50 g of styrene oxide over 20 minutes. The resulting mixture was stirred for 2 hours at the same temperature. The reaction mixture was added to a mixture of

30 ml of diethyl ether and 30 ml of ice water. The organic layer was separated and mixed with 10 ml of water. The mixture was adjusted to pH 2.0 with 6 N hydrochloric acid. The aqueous layer was separated and mixed with 30 ml of diethyl ether. The mixture was adjusted to pH 11 with a 2 N aqueous sodium hydroxide solution. The organic layer was separated, washed with a saturated aqueous sodium chloride solution, and dried over anhydrous magnesium sulfate. The solvent was removed by distillation under reduced pressure. The residue was purified by column chromatography (eluant: toluene/ethyl acetate = 1/1) to obtain 2.00 g of oily 1-phenyl-2-[(4-benzylmorpholin-2-yl)methoxy]ethanol (compound No. 144).

The compounds shown in Table 7 were obtained in the same manner.

In Table 7 $R^1$, $R^2$, $R^3$, $R^4$, $R^6$ and n each show a substituent or integer used in the following formula:

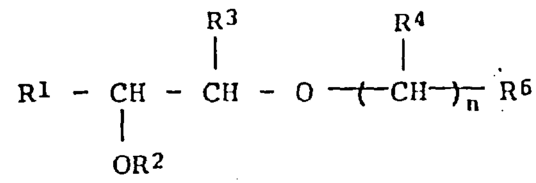

Table 7

| Compound No. | R1 | R2 | R3 | R4 | R6 | n | Addition salt | Melting point (°C) |
|---|---|---|---|---|---|---|---|---|
| 145 | 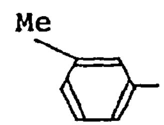 | H | H | H | 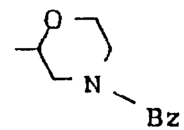 | 1 | - | Oily |
| 146 | 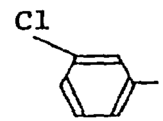 | " | " | " | " | " | " | " |
| 147 | 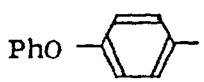 | " | " | " | " | " | " | " |
| 148 | 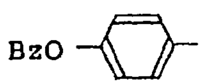 | " | " | " | 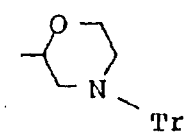 | " | " | " |

- Cont'd -

Table 7 (Cont'd)

| | | | | | | | | |
|---|---|---|---|---|---|---|---|---|
| 149 | 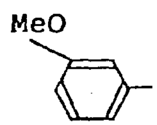 | H | H | H | 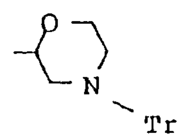 | 1 | - | Oily |
| 150 | 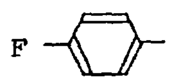 | " | " | " | " | " | " | " |

(2) A mixture of 1.00 g of 1-phenyl-2-[(4-benzylmorpholin-2-yl)methoxy]ethanol, 500 mg of 5% palladium-carbon and 10 ml of methanol was subjected to hydrogenation for 4 hours at room temperature under atmospheric pressure. After the completion of the reaction, palladium-carbon was removed by filtration. The solvent was removed by distillation under reduced pressure. The residue was purified by column chromatography (eluant: chloroform/methanol = 10/1) to obtain 450 mg of an oily product. The oily product was dissolved in 2.5 ml of isopropanol. The solution was mixed with 220 mg of fumaric acid. The mixture was stirred for 1 hour at room temperature. The resulting crystals were collected by filtration, washed with 2 ml of isopropanol and dried to obtain 460 mg of 1/2 fumarate of 1-phenyl-2-[(morpholin-2-yl)methoxy]ethanol (compound No. 151).

Melting point: 146.5-147°C [EtOH]

The compounds shown in Table 8 were obtained in the same manner.

In Table 8, $R^1$, $R^2$, $R^3$, $R^4$, $R^6$ and n each show a substituent or integer used in the following formula:

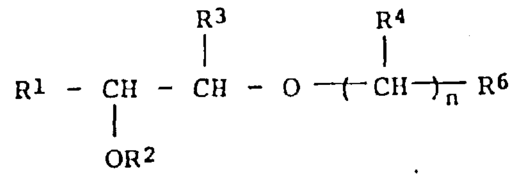

Table 8

| Compound No. | $R^1$ | $R^2$ | $R^3$ | $R^4$ | $R^6$ | n | Addition salt | Melting point (°C) |
|---|---|---|---|---|---|---|---|---|
| 152 | 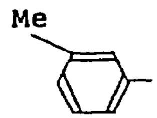 | H | H | H | 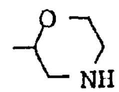 | 1 | 1/2 Fumaric acid | 155-156 [EtOH] |
| 153 | 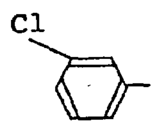 | " | " | " | " | " | HCl | Amorphous |
| 154 | 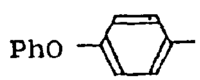 | " | " | " | " | " | 1/2 Fumaric acid | 142-142.5 [EtOH-$Me_2CO$] |

(3) 7 g of 1-(3-methoxyphenyl)-2-[(4-trityl-morpholin-2-yl)methoxy]ethanol was dissolved in 35 ml of acetone. To the solution was added 2.6 ml of a 5.9 N dry hydrogen chloride-ethanol solution with ice cooling. The mixture was stirred for 2 hours at room temperature. After the completion of the reaction, the solvent was removed by distillation under reduced pressure. To the residue thus obtained were added 50 ml of water and 30 ml of ethyl acetate. The aqueous layer was separated and washed with ethyl acetate. Thereto was added 50 ml of chloroform. The resulting mixture was adjusted to pH 11 with a 1 N aqueous sodium hydroxide solution. The organic layer was separated and dried over anhydrous magnesium sulfate. The solvent was removed by distillation under reduced pressure. The residue thus obtained was purified by column chromatography (eluant: chloroform/methanol = 5/1) to obtain 1 g of an oily product. The oily product was dissolved in 3 ml of isopropanol. Thereto was added 430 mg of fumaric acid. The mixture was stirred for 1 hour at room temperature. The resulting crystals were collected by filtration and dried to obtain 800 mg of 1/2 fumarate of 1-(3-methoxyphenyl)-2-[(morpholin-2-yl)methoxy]ethanol (compound No. 155).

Melting point: 122-123.5°C [EtOH]

The compounds shown in Table 9 were obtained in the same manner.

In Table 9, $R^1$, $R^2$, $R^3$, $R^4$, $R^6$ and n each show a substituent or integer used in the following formula:

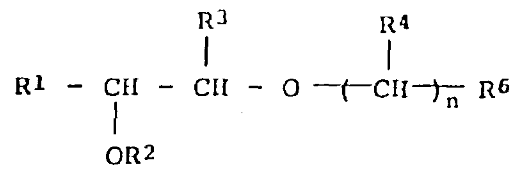

Table 9

| Compound No. | $R^1$ | $R^2$ | $R^3$ | $R^4$ | $R^6$ | n | Addition salt | Melting point (°C) |
|---|---|---|---|---|---|---|---|---|
| 156 | 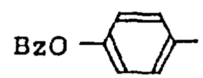 | H | H | H | 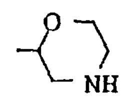 | 1 | 1/2 Fumaric acid | 147-148.5 [EtOH] |
| 157 | 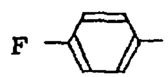 | " | " | " | " | " | " | 126-127 [IPA] |

Production Example 6 (Reference)

A mixture of 2.0 g of 1-(4-benzyloxyphenyl)-2-[2-(N,N-dimethylamino)ethoxy]ethanol hydrochloride, 500 mg of 10% palladium-carbon and 10 ml of methanol was subjected to hydrogenation for 2 hours at room temperature under atmospheric pressure. After the completion of the reaction, palladium-carbon was removed by filtration. The solvent was removed by distillation under reduced pressure to obtain 1.4 g of 1-(4-hydroxyphenyl)-2-[2-(N,N-dimethylamino) ethoxy]ethanol hydrochloride

Melting point: 169.5-170.5°C [EtOH]

The compounds shown in Table 10 were obtained in the same manner.

In Table 10, $R^1$, $R^2$, $R^3$, $R^4$, $R^6$ and n each show a substituent or integer used in the following formula:

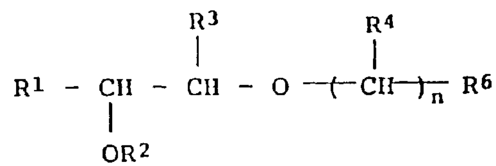

Table 10

| Compound No. | $R^1$ | $R^2$ | $R^3$ | $R^4$ | $R^6$ | n | Addition salt | Melting point (°C) |
|---|---|---|---|---|---|---|---|---|
| 176 | 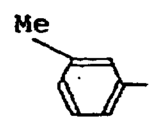 | H | H | H | 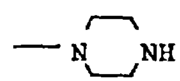 | 2 | - | Oily |

Production Example 7 (Reference)

0.65 ml of pyridine and 0.99 ml of acetic anhydride were added to 1 g of 1-(3-methylphenyl)-2-[2-(morpholin-4-yl) ethoxy]ethanol. The mixture was stirred for 3 hours at room temperature. The reaction mixture was subjected to distillation under reduced pressure to remove the solvent. To the residue were added 20 ml of ethyl acetate and 10 ml of water. The mixture was adjusted to pH 10 with potassium carbonate. The organic layer was separated, washed with water and a saturated aqueous sodium chloride solution in this order, and dried over anhydrous magnesium sulfate. The solvent was removed by distillation under reduced pressure. The residue was purified by column chromatography (eluant: chloroform/ethanol = 30/l) to obtain 1 g of oily 1-acetoxy-1-(3-methylphenyl)-2-[2-(morpholin-4-yl)ethoxy] ethane.

The compound shown in Table 11 was obtained in the same manner.

In Table 11 $R^1$, $R^2$, $R^3$, $R^4$, $R^6$ and n each show a substituent or integer used in the following formula:

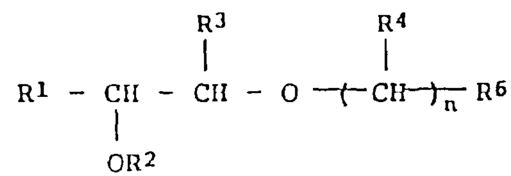

Table 11

| Compound No. | $R^1$ | $R^2$ | $R^3$ | $R^4$ | $R^6$ | n | Addition salt | Melting point (°C) |
|---|---|---|---|---|---|---|---|---|
| 188 | 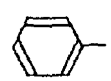 | Ac | H | H | 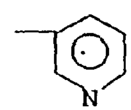 | 1 | HCl | Oily |

Production Example 8

A mixture of 500 mg of 2-[(imidazol-4-yl)methoxy]-1-phenylethanol, 1.1 ml of pyridine, 1.1 ml of triethylamine and 1.1 ml of acetic anhydride was stirred for 1 hour at 100°C. The reaction mixture was cooled to room temperature. The solvent was removed by distillation under reduced pressure. To the residue were added 1.1 ml of methyl iodide and 5 ml of acetonitrile. The mixture was allowed to stand at room temperature for 24 hours. The solvent was removed by distilation under reduced pressure. To the residue thus obtained were added 4 ml of ethanol and 6.8 ml of a 5% aqueous sodium hydroxide solution. The resulting mixture was stirred at room temperature for 6 hour. The solvent was removed by distillation under reduced pressure. To the residue thus obtained were added 30 ml of chloroform and 20 ml of water. The organic layer was separatd, washed with water, and dried over anhydrous magnesium sulfate. The solvent was removed by distillation under reduced pressure. The resulting oily product was purified by column chromatography (eluant: chloroform/ethanol = 10/1). Diisopropyl ether was added to the resulting white crystals, and the resulting mixture was filtered to obtainn 450 mg of 2-[(1-methylimidazol-5-yl)methoxy]-l-phenylethanol (compound No. 214).

Melting point: 102-105°C

The following compound was obtained in the same manner.
1-(4-Benzyloxyphenyl)-2-[(1-methylimidazol-5-yl)-methoxy]ethanol (compound No. 215)

Melting point: 148.5-150.5°C [IPA-AcOEt]

Production Example 9

(1) 5 g of (S)-4-benzyl-2-acetoxymethylmorpholine was dissolved in 10 ml of ethanol. To the solution was added a solution of 1 g of sodium hydroxide dissolved in 5 ml of water with ice cooling. The mixture was stirred at room temperature for 10 minutes. The reaction mixture was added to 50 ml of ice water and extracted with 50 ml of chloroform. The organic layer was washed with water and a saturated aqueous sodium chloride solution in this order, and dried over anhydrous magnesium sulfate. The solvent was removed by distillation under reduced pressure. The residue thus obtained was purified by column chromatography (eluant: chloroform/ethanol = 30/1) to obtain 3.6 g of oily (S)-4-benzyl-2 -hydroxymethylmorpholine.

(2) 3.5 g of (5)-4-benzyl-2-hydroxymethyl-morpholine was dissolved in 5 ml of ethanol. To the solution was added 5 ml of a 5.9 N hydrogen chloride-ethanol solution with ice cooling. The resulting mixture was stirred for 10 minutes at the same temperature. Thereto was added a mixture of 500 mg of 5% palladium-carbon and 10 ml of methanol. The resulting mixture was subjected to hydrogenation for 3 hours at 40°C. After the completion of the reaction, palladium-carbon was removed by filtration. The solvent was removed by distillation under reduced pressure to obtain a yellow oily product. To the oily product were added 20 ml of dry methylene chloride and 4.7 ml of triethylamine. The resulting mixture was stirred at room temperature for 30 minutes. Thereto was dropwise added a solution of 4.7 g of trityl chloride dissolved in 10 ml of methylene chloride in 20 minutes, with ice cooling. The mixture was stirred at room temperature for 3 hours and added to 50 ml of ice water. The organic layer was separated and 20 ml of water was added thereto. The resulting mixture was adjusted to pH 12 with a 1 N aqueous sodium hydroxide solution. The organic layer was separated and dried over anhydrous magnesium sulfate. The solvent was removed by distillation under reduced pressure to obtain a yellow oily product. The oily product was mixed with 10 ml of diisopropyl ether. The resulting crystals were collected by filtration to obtain 2.7 g of (S)-4-trityl-2-hydroxymethylmorpholine.

Melting point: 142.0-142.5°C [AcOEt-IPE]
Optical rotation: $[\alpha]_D^{27}$ = -10.9° (C=l, $CHCl_3$)

The (2R)-form having the following properties was obtained in the same manner.

Melting point: 142.0-142.5°C [AcOEt-IPE]
Optical rotation: $[\alpha]_D^{27}$ = +10.9° (C=l, $CHCl_3$)

(3) The same procedure as in (1) of Production Example 5 was repeated, except that the styrene oxide and the 4-benzyl-2-hydroxymethylmorpholine were replaced by (S)-2-phenyloxirane and (S)-4-trityl-2-hydroxy-methylmorpholine, respectively, to obtain oily (1S,2'S)-1-phenyl-2-[(4-tritylmorpholin-2-yl)methoxy]ethanol (compound No. 217).

(4) In 20 ml of acetone was dissolved (1S,2'S)-1-phenyl-2-[(4-tritylmorpholin-2-yl)methoxy]ethanol. To the solution

was added, with ice cooling, 7 ml of a 5.9 N dry hydrogen chloride-ethanol solution. The resulting mixture was stirred for 30 minutes at room temperature to effect a reaction. After the completion of the reaction, the solvent was removed by distillation under reduced pressure. The residue thus obtained was added to a mixture of 30 ml of ice water and 30 ml of ethyl acetate. The aqueous layer was separated and washed with 30 ml of ethyl acetate,-after which 50 ml of chloroform was added thereto. The resulting mixture was adjusted to pH 11 with a 2 N aqueous sodium hydroxide solution. The organic layer was separated, washed with a saturated aqueous sodium chloride solution, and dried over anhydrous magnesium sulfate. The solvent was removed by distillation under reduced pressure to obtain 1.9 g of a yellow oily product. The oily product was dissolved in 10 ml of ethanol. To the solution was added 720 mg of oxalic acid. The resulting mixture was heated to obtain a solution. The solution was allowed to stand overnight at room temperature. The resulting crystals were collected by filtration and dried to obtain 1.8 g of (1S,2'S)-1-phenyl-2-[(morpholin-2-yl)methoxy]ethanol oxalate (compound No. 218).

Melting point: 130-132°C [EtOH]
Optical rotation: $[\alpha]_D^{24}$ = +19.9° (C=l, $CHCl_3$)

The following compounds were obtained in the same manner.
(1R,2'S)-1-phenyl-2-[(morpholin-2-yl)methoxy]ethanol maleate (compound No. 219)

Melting point: 136-136.5°C [EtOH]
Optical rotation: $[\alpha]_D^{25}$ = -21.7° (C=l, $CHCl_3$)

(1S,2'R)-1-phenyl-2-[(morpholin-2-yl)methoxy]ethanol maleate (compound No. 220)

Melting point: 136-136.5°C [EtOH]
Optical rotation: $[\alpha]_D^{25}$ = +22.2° (C=l, $CH_3OH$)

(1R,2'R)-1-phenyl-2-[(morpholin-2-yl)methoxy]ethanol oxalate (compound No. 221)

Melting point: 131-132.5°C [EtOH]
Optical rotation: $[\alpha]_D^{25}$= -20.4° (C=l, $CH_3OH$)

Next, this invention is specifically described by way of Examples. However, this invention is in no way restricted to these Examples.

Example 1 (Tablets) (Reference)

Tablets each containing 50 mg of 1-(4-benzyloxyphenyl)-2-[2-(N,N-dimethylamino)ethoxy]ethanol hydrochloride (compound No. 48) were prepared using the following recipe according to the following method:

Per tablet:

| | | |
|---|---|---|
| Compound No. 48 | 50 mg | ① |
| Lactose | 20 mg | ① |
| Kollidon CL (BASF's product) | 15 mg | ① |
| Corn starch | 30 mg | ① |
| AVICEL PH 101 (Asahi Kasei's product) | 50 mg | ① |
| Polyvinylpyrrolidone K-90 | 5 mg | |
| Light silica | 3 mg | ② |
| Magnesium stearate | 2 mg | ② |
| Total | 175 mg | |

The components ① were kneaded with an aqueous solution containing 8% of Polyvinylpyrrolidone K-90. The kneaded product was dried at 40°C and mixed with the components ②. The resulting mixture was made into round tablest each weighing 175 mg and having a diameter of 8 mm.

Example 2 (Capsules) (Reference)

Capsules each containing 50 mg of 2-[2-(N,N-dimethylamino) ethoxy]-1-[4-(4-phenyloxy)phenyl]ethanol hydrochloride (compound No. 54) were prepared using the following recipe according to the following method:

| Per capsule: | | |
|---|---|---|
| Compound No. 54 | 50 mg | ① |
| Lactose | 20 mg | ① |
| Corn starch | 53 mg | ① |
| Kollidon CL (BASF's product) | 2 mg | ① |
| Polyvinylpyrrolidone K-90 | 5 mg | |
| AVICEL PH 302 (Asahi Kasei's product) | 18 mg | ② |
| Magnesium stearate | 2 mg | ② |
| Total | 150 mg | |

The components①were kneaded with an aqueous solution containing 8% of Polyvinylpyrrolidone K-90. The kneaded product was dried at 40°C and mixed with the components② The resulting mixture was charged into No. 3 gelatin capsules in an amount of 150 mg per capsule to obtain capsules.

Example 3 (Liquid) (Reference)

A liquid containing 25 mg of 2-[2-(N,N-dimethylamino)ethoxy]-1-(3-trifluoromethylphenyl)ethanol hydrochloride (compound No. 42) was prepared using the following recipe according to the following method:

| Per ampule: | |
|---|---|
| Compound No. 42 | 25 mg |
| Methyl paraoxybenzoate | 1 mg |
| Total | 26 mg |

The above components were dissolved in physiological saline and the total volume of the solution was made into 1 ml. The solution was filtered aseptically and charged into an ampule to obtain a liquid.

Example 4 (Injection) (Reference)

An injection containing 25 mg of 2-[2-(N,N-dimethylamino)ethoxy]-1-(3-fluorophenyl)ethanol hydrochloride (compound No. 1) was prepared using the following recipe according to the following method:

| | |
|---|---|
| Compound No. 1 | 25 mg |
| Manitol | 75 mg |
| Total | 100 mg |

The above components were dissolved in 1.5 ml of distilled water prepared for injection. The solution was filtered aseptically, charged into a 3-ml minivial, and freeze-dried to obtain an injection.

Example 5 (fine granules) (Reference)

Fine granules each containing 50 mg of 2-(1-benzylpiperidin-4-yloxy)-1-phenylethanol hydrochloride (compound No. 112) were prepared using the following recipe according to the following method:

| | | |
|---|---|---|
| Compound No. 112 | 50 mg | ① |
| α-Starch | 200 mg | ① |
| Purified sucrose | 250 mg | ① |
| Lactose | 470 mg | ① |
| Polyvinylpyrrolidone K-90 | 30 mg | |
| Total | 1000 mg | |

The components ① were subjected to granulation under high speed stirring with an aqueous solution containing 8% of Polyvinylpyrrolidone K-90. The granules obtained were sieved through a 32-mesh screen and dried to obtain fine granules.

Example 6 (Tablets)

2-[(1-methylimidazol-5-yl)methoxy]-1-(4-benzyloxyphenyl)-ethanol (compound No. 215), were processed as in Example 1 to obtain tablets each containing 50 mg of one of the above compounds.

Example 7 (Capsules)

2-[(1-methylimidazol-5-yl)methoxy]-1-(4-benzyloxyphenyl)-ethanol (compound No. 215), were processed as in Example 2 to obtain capsules each containing 50 mg of one of the above compounds.

## Claims

**1.** A 1,2-ethanediol derivative represented by the following general formula or a salt thereof:

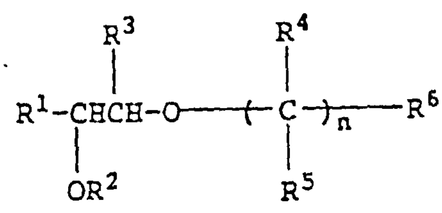

wherein $R^1$ represents a substituted or unsubstituted phenyl group; $R^2$ represents a hydrogen atom, a $C_{1-6}$ alkyl group or a hydroxyl-protecting group; $R^3$ represents a hydrogen atom or a $C_{1-6}$ alkyl group; $nR^4$'s and $nR^5$'s are the same as or different from one another and represent hydrogen atoms or $C_{1-6}$ alkyl groups; n represents an integer of 1 to 6; and $R^6$ represents a substituted or unsubstituted nitrogen-containing heterocyclic group, the nitrogen-containing heterocyclic group attaching to carbon atom through a carbon atom forming the heterocyclic ring and being selected when n represents 1 from the group consisting of pyrrolyl, pyrrolidinyl, piperazinyl, imidazolyl, pyridyl, tetrahydropyridyl, pyrimidinyl, morpholinyl, thiomorpholinyl, quinolyl, quinolizinyl, tetrahydroquinolinyl, tetrahydroisoquinolinyl, quinuclidinyl, thiazolyl, tetrazolyl, thiadiazolyl, pyrrolinyl, imidazolinyl, imidazolidinyl, pyrazolinyl, pyrazolidinyl, purinyl and indazolyl groups or being selected when n represents an integer of 2 to 6 from the group consisting of pyrrolyl, pyrrolidinyl, piperidyl, piperazinyl, imidazolyl, pyridyl, tetrahydropyridyl, pyrimidinyl, morpholinyl, thiomorpholinyl, quinolyl, quinolizinyl, tetrahydroquinolinyl, tetrahydroisoquinolinyl, quinuclidinyl, thiazolyl, tetrazolyl, thiadiazolyl, pyrrolinyl, imidazolinyl, imidazolidinyl, pyrazolinyl, pyrazolidinyl, purinyl and indazolyl groups, wherein the substituent on $R^1$ is selected from the group consisting of halogen atoms, substituted or unsubstituted amino, $C_{1-6}$ alkyl, aryl, ar-$C_{1-4}$ alkyl, $C_{1-6}$ alkoxy, ar-$C_{1-4}$ alkoxy, aryloxy, carbamoyloxy, $C_{1-6}$ alkylthio, $C_{2-6}$ alkenyl, $C_{2-6}$ alkenyloxy, ar-$C_{1-4}$ alkylthio, ar-$C_{1-6}$ alkylsulfonyl, arylsulfonyl, $C_{1-6}$ alkylsulfonylamino, arylsulfonylamino and heterocyclic groups, protected amino groups, protected or unprotected hydroxyl groups, nitro group, oxo group and $C_{1-4}$ alkylenedioxy groups; the substituted $C_{1-6}$ alkyl, aryl, ar-$C_{1-4}$ alkyl, $C_{1-6}$ alkoxy, ar-$C_{1-4}$ alkoxy, aryloxy, carbamoyloxy, $C_{1-6}$ alkylthio, $C_{2-6}$ alkenyl, $C_{2-6}$ alkenyloxy, ar-$C_{1-4}$ alkylthio, ar-$C_{1-6}$ alkylsulfonyl, arylsulfonyl, $C_{1-6}$ alkylsulfonylamino, arylsulfonylamino or heterocyclic group as the substituent of $R^1$ and the substituted nitrogen-containing heterocyclic group as $R^6$ have each at least one substituent selected from the group consisting of halogen atoms, protected or unprotected hydroxyl groups, protected or unprotected amino groups, protected or unprotected carboxyl groups, unsubstituted $C_{1-6}$ alkyl groups, $C_{1-6}$ alkyl groups substituted by a protected or unprotected hydroxyl group, unsubstituted or halogen-substituted aryl groups, unsubstituted or halogen-substituted aroyl groups, unsubstituted $C_{1-6}$ alkoxy groups, $C_{1-6}$ alkoxy groups substituted by a $C_{1-6}$ alkoxy group, $C_{1-6}$ acyl groups, ar-$C_{1-4}$ alkyl groups, ar-$C_{2-6}$ alkenyl groups, heterocyclic groups, heterocyclic-CO-groups, oxo group, $C_{1-6}$ alkylsulfonyl groups and arylsulfonyl groups; and the substituted amino group as the substituent of $R^1$ has each at least one substituent selected from the group consisting of protected or unprotected hydroxyl groups, unsubstituted $C_{1-6}$ alkyl groups, $C_{1-6}$ alkyl groups substituted by a protected or unprotected carboxyl or hydroxyl group, cycloalkyl groups, aryl groups, $C_{1-6}$ acyl groups, ar-$C_{1-4}$ alkyl groups, heterocyclic groups, unsubstituted or oxo-substituted heterocyclic-CO- groups, adamantyl group, $C_{1-6}$ alkylsulfonyl groups and arylsulfonyl groups; all the above heterocyclic groups being selected from the group consisting of the nitrogen-containing heterocyclic groups mentioned in the definition of $R^6$ and furyl, thienyl, benzothienyl, pyranyl, isobenzofuranyl, oxazolyl, benzofuranyl, indolyl, benzimidazolyl, benzoxazolyl, benzothiazolyl, quinoxalyl, dihydroquinoxalyl, 2,3-dihydrobenzothienyl, 2,3-dihydrobenzopyrrolyl, 2,3-dihydro-4H-1-thianaphthyl, 2,3-dihydrobenzofuranyl, benzo[b]dioxanyl, imidazo[2,3-a]pyridyl, benzo[b]piperazinyl, chromenyl, isothiazolyl, isoxazolyl, oxadiazolyl, pyridazinyl, isoindolyl and isoquinolyl groups.

2. A 1,2-ethanediol derivative or a salt thereof according to Claim 1, wherein $R^2$ represents a hydrogen atom or a hydroxyl-protecting group; $nR^4$'s are the same as or different from one another and represent hydrogen atoms or $C_{1-6}$ alkyl groups; $nR^5$'s represent hydrogen atoms; and $R^6$ represents a substituted or unsubstituted nitrogen-containing heterocyclic group, the nitrogen-containing heterocyclic group attaching to carbon atom through a carbon atom forming the heterocyclic ring and being selected from the group consisting of pyrrolyl, piperazinyl, imidazolyl, pyridyl, pyrimidinyl, quinolyl, quinolizinyl, tetrahydroquinolinyl, quinuclidinyl, thiazolyl and thiadiazolyl groups.

3. 1-(4-Benzyloxyphenyl)-2- [(1-methyl-5-imidazolyl) methoxy]ethanol or a salt thereof.

4. Use of a 1,2-ethanediol derivative represented by the following general formula or a salt thereof for preparing a drug for treating cerebrovascular dementia, senile dementia, Alzheimer's dementia, sequelae of ischemic encephalopathy or cerebral apoplexy in a patient:

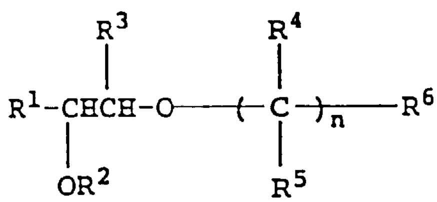

wherein $R^1$ represents a substituted or unsubstituted phenyl group; $R^2$ represents a hydrogen atom, a $C_{1-6}$ alkyl group or a hydroxyl-protecting group; $R^3$ represents a hydrogen atom or a $C_{1-6}$ alkyl group; $nR^4$'s and $nR^5$'s are the same as or different from one another and represent hydrogen atoms or $C_{1-6}$ alkyl groups; $R^6$ represents a substituted or unsubstituted nitrogen-containing heterocyclic group, the nitrogen-containing heterocyclic group

attaching to oxygen atom when n is zero or to carbon atom when n is an integer of 1 to 6 through a carbon atom forming the heterocyclic ring and being selected from the group consisting of pyrrolyl, pyrrolidinyl, piperidyl, piperazinyl, imidazolyl, pyridyl, tetrahydropyridyl, pyrimidinyl, morpholinyl, thiomorpholinyl, quinolyl, quinolizinyl, tetrahydroquinolinyl, tetrahydroisoquinolinyl, quinuclidinyl, thiazolyl, tetrazolyl, thiadiazolyl, pyrrolinyl, imidazolinyl, imidazolidinyl, pyrazolinyl, pyrazolidinyl, purinyl and indazolyl groups; and n represents 0 or an integer of 1 to 6, wherein the substituent on $R^1$ is selected from the group consisting of halogen atoms, substituted or unsubstituted amino, $C_{1-6}$ alkyl, aryl, ar-$C_{1-4}$ alkyl, $C_{1-6}$ alkoxy, ar-$C_{1-4}$ alkoxy, aryloxy, carbamoyloxy, $C_{1-6}$ alkylthio, $C_{2-6}$ alkenyl, $C_{2-6}$ alkenyloxy, ar-$C_{1-4}$ alkylthio, ar-$C_{1-6}$ alkylsulfonyl, arylsulfonyl, $C_{1-6}$ alkylsulfonylamino, arylsulfonylamino and heterocyclic groups, protected amino groups, protected or unprotected hydroxyl groups, nitro group, oxo group and $C_{1-4}$ alkylenedioxy groups; the substituted $C_{1-6}$ alkyl, aryl, ar-$C_{1-4}$ alkyl, $C_{1-6}$ alkoxy, ar-$C_{1-4}$ alkoxy, aryloxy, carbamoyloxy, $C_{1-6}$ alkylthio, $C_{2-6}$ alkenyl, $C_{2-6}$ alkenyloxy, ar-$C_{1-4}$ alkylthio, ar-$C_{1-6}$ alkylsulfonyl, arylsulfonyl, $C_{1-6}$ alkylsulfonylamino, arylsulfonylamino or heterocyclic group as the substituent of $R^1$ and the substituted nitrogen-containing heterocyclic group as $R^6$ have each at least one substituent selected from the group consisting of halogen atoms, protected or unprotected hydroxyl groups, protected or unprotected amino groups, protected or unprotected carboxyl groups, unsubstituted $C_{1-6}$ alkyl groups, $C_{1-6}$ alkyl groups substituted by a protected or unprotected hydroxyl group, unsubstituted or halogen-substituted aryl groups, unsubstituted or halogen-substituted aroyl groups, unsubstituted $C_{1-6}$ alkoxy groups, $C_{1-6}$ alkoxy groups substituted by a $C_{1-6}$ alkoxy group, $C_{1-6}$ acyl groups, ar-$C_{1-4}$ alkyl groups, ar-$C_{2-6}$ alkenyl groups, heterocyclic groups, heterocyclic-CO-groups, oxo group, $C_{1-6}$ alkylsulfonyl groups and arylsulfonyl groups; and the substituted amino group as the substituent of $R^1$ has each at least one substituent selected from the group consisting of protected or unprotected hydroxyl groups, unsubstituted $C_{1-6}$ alkyl groups, $C_{1-6}$ alkyl groups substituted by a protected or unprotected carboxyl or hydroxyl group, cycloalkyl groups, aryl groups, $C_{1-6}$ acyl groups, ar-$C_{1-4}$ alkyl groups, heterocyclic groups, unsubstituted or oxo-substituted heterocyclic-CO-groups, adamantyl group, $C_{1-6}$ alkylsulfonyl groups and arylsulfonyl groups; all the above heterocyclic groups being selected from the group consisting of the nitrogen-containing heterocyclic groups mentioned in the definition of $R^6$ and furyl, thienyl, benzothienyl, pyranyl, isobenzofuranyl, oxazolyl, benzofuranyl, indolyl, benzimidazolyl, benzoxazolyl, benzothiazolyl, quinoxalyl, dihydroquinoxalyl, 2,3-dihydrobenzothienyl, 2,3-dihydrobenzopyrrolyl, 2,3-dihydro-4H-1-thianaphthyl, 2,3-dihydrobenzofuranyl, benzo [b] dioxanyl, imidazo [2,3-a] pyridyl, benzo [b] piperazinyl, chromenyl, isothiazolyl, isoxazolyl, oxadiazolyl, pyridazinyl, isoindolyl and isoquinolyl groups.

**5.** Use of a 1,2-ethanediol derivative or a salt thereof according to Claim 4, wherein $R^2$ represents a hydrogen atom or a hydroxyl-protecting group; $nR^{4}$'s are the same as or different from one another and represent hydrogen atoms or $C_{1-6}$ alkyl groups; $nR^{5}$'s represent hydrogen atoms; and $R^6$ represents a substituted or unsubstituted nitrogen-containing heterocyclic group, the nitrogen-containing heterocyclic group attaching to oxygen atom when n is zero or to carbon atom when n is an integer of 1 to 6 through a carbon atom forming the heterocyclic ring and being selected from the group consisting of pyrrolyl, pyrrolidinyl, piperidinyl, piperazinyl, imidazolyl, pyridyl, pyrimidinyl, morpholinyl, quinolyl, quinolizinyl, tetrahydroquinolinyl, quinuclidinyl, thiazolyl and thiadiazolyl groups.

**6.** Use of a 1,2-ethanediol derivative or a salt thereof according to Claim 4, wherein $R^6$ represents a substituted or unsubstituted nitrogen-containing heterocyclic group, the nitrogen-containing heterocyclic group attaching to oxygen atom when n is zero or to carbon atom when n is an integer of 1 to 6 through a carbon atom forming the heterocyclic ring and being selected from the group consisting or pyrrolinyl, piperidyl, piperazinyl, imidazolyl, pyridyl, morpholinyl, thiomorpholinyl, tetrahydropyridyl, quinuclidinyl and tetrazolyl, the substituent on $R^1$ is selected from the group consisting of halogen atoms; a $C_{1-6}$ alkyl groups; $C_{2-6}$ alkenyl groups; phenyl groups which may optionally be substituted by a halogen atom or a $C_{1-6}$ alkyl or $C_{1-6}$ alkoxy group; phenyl-$C_{1-4}$ alkyl groups; $C_{1-6}$ alkoxy groups which may optionally be substituted by a pyridyl, furyl or thienyl group; phenyl-$C_{1-4}$ alkoxy groups which may optionally be substituted by a halogen atom or a $C_{1-6}$ alkyl, hydroxyl or $C_{1-6}$ alkoxy group; a phenoxy group; phenylaminocarbonyloxy groups which may optionally be substituted by a halogen atom; $C_{1-6}$ alkylaminocarbonyloxy groups; $C_{1-6}$ alkylthio groups; a phenylsulfonylamino group; amino groups which may optionally be substituted by a $C_{1-6}$ alkyl group; a hydroxyl group; a nitro group; an oxo group; phenyl-$C_{1-4}$ alkylthio groups; phenyl-$C_{1-4}$ alkylsulfonyl groups; a thienyl group; a pyridyl group; and $C_{1-4}$ alkylenedioxy groups; and the substituent on $R^6$ is selected from the group consisting of halogen atoms; a hydroxyl group; $C_{1-6}$ alkyl groups which may optionally be substituted by a hydroxyl group; an amino group; $C_{1-6}$ acyl groups which may optionally be substituted by a pyrrolidinyl group; phenyl-$C_{1-4}$ alkyl groups; phenyl-$C_{2-4}$ alkenyl groups; aroyl groups which are substituted by a halogen atom; a pyridyl group and an oxo group.

**7.** Use of a 1,2 -ethanediol derivative or a salt thereof according to Claim 6, wherein $R^1$ represents a phenyl group which may optionally be substituted by a halogen atom; a phenyl-$C_{1-4}$ alkoxy group; a phenyl group; a thienyl

group; a phenoxy group which may optionally be substituted by a halogen atom or a $C_{1-6}$ alkoxy group; or a $C_{1-6}$ alkoxy group which may optionally be substituted by a thienyl group; $R^2$ represents a hydrogen atom; $R^3$ represents a hydrogen atom; $nR^4$'s and $nR^5$'s represent hydrogen atoms; $R^6$ represents a morpholinyl group, a pyridyl group or an imidazolyl group which may optionally be substituted by a $C_{1-6}$ alkyl group.

**8.** Use of a 1,2-ethanediol derivative or a salt thereof according to Claim 4, wherein the derivative or salt is 1-(4-benzyloxyphenyl)-2-[(1-methyl-5-imidazolyl)methoxy]ethanol or a salt thereof.

**9.** A cerebral function-improving agent comprising a 1,2-ethanediol derivative represented by the following general formula or a salt thereof:

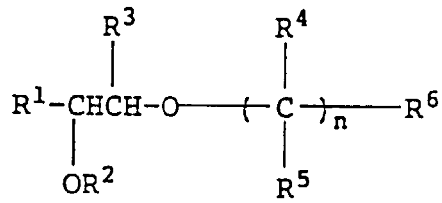

wherein $R^1$, $R^2$, $R^3$, $R^4$, $R^5$, $R^6$ and n are as defined in claim 4.

## Patentansprüche

**1.** 1,2-Ethandiol-Derivat, dargestellt durch die folgende allgemeine Formel, oder ein Salz desselben:

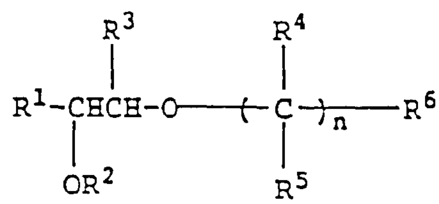

in der $R^1$ eine substituierte oder unsubstituierte Phenylgruppe darstellt; $R^2$ ein Wasserstoffatom, eine $C_{1-6}$-Alkylgruppe oder eine Hydroxyl-Schutzgruppe darstellt; $R^3$ ein Wasserstoffatom oder eine $C_{1-6}$-Alkyl-gruppe darstellt; die $nR^4$'s und $nR^5$'s gleich oder verschieden voneinander sind und Wasserstoffatome oder $C_{1-6}$-Alkylgruppen darstellen; n eine ganze Zahl von 1 bis 6 darstellt; und $R^6$ eine substituierte oder unsubstituierte stickstoffhaltige heterocyclische Gruppe darstellt, wobei die stickstoffhaltige heterocyclische Gruppe am Kohlenstoffatom durch ein Kohlenstoffatom, das den heterocyclischen Ring bildet, angebracht ist und, wenn n 1 darstellt, aus der Gruppe ausgewählt ist, die aus Pyrrolyl-, Pyrrolidinyl-, Piperazinyl-, Imidazolyl-, Pyridyl-, Tetrahydropyridyl-, Pyrimidinyl-, Morpholinyl-, Thiomorpholinyl-, Chinolyl-, Chinolizinyl-, Tetrahydrochinolinyl-, Tetrahydroisochinolinyl-, Chinuclidinyl-, Thiazolyl-, Tetrazolyl-, Thiadiazolyl-, Pyrrolinyl-, Imidazolinyl-, Imidazolidinyl-, Pyrazolinyl-, Pyrazolidinyl-, Purinyl- und Indazolylgruppen besteht, oder, wenn n eine ganze Zahl von 2 bis 6 darstellt, aus der Gruppe ausgewählt ist, die aus Pyrrolyl-, Pyrrolidinyl-, Piperidyl-, Piperazinyl-, Imidazolyl-, Pyridyl-, Tetrahydropyridyl-, Pyrimidinyl-, Morpholinyl-, Thiomorpholinyl-, Chinolyl-, Chinolizinyl-, Tetrahydrochinolinyl-, Tetrahydroisochinolinyl-, Chinuclidinyl-, Thiazolyl-, Tetrazolyl-, Thiadiazolyl-, Pyrrolinyl-, Imidazolinyl-, Imidazolidinyl-, Pyrazolinyl-, Pyrazolidinyl-, Purinyl- und Indazolylgruppen besteht, worin der Substituent an $R^1$ aus der Gruppe ausgewählt ist, die aus Halogenatomen, substituierten oder unsubstituierten Amino-, $C_{1-6}$-Alkyl-, Aryl-, Ar-$C_{1-4}$-alkyl-, $C_{1-6}$-Alkoxy-, Ar-$C_{1-4}$-alkoxy-, Aryloxy-, Carbamoyloxy-, $C_{1-6}$-Alkythio-, $C_{2-6}$-Alkenyl-, $C_{2-6}$-Alkenyloxy-, Ar-$C_{1-4}$-alkylthio-, Ar-$C_{1-6}$-alkylsulfonyl-, Arylsulfonyl-, $C_{1-6}$-Alkylsulfonylamino-, Arylsulfonylamino-und heterocyclischen Gruppen, geschützten Aminogruppen, geschützten oder ungeschützten Hydroxylgruppen, der Nitrogruppe, Oxogruppe und $C_{1-4}$-Alky-

lendioxygruppen besteht; die substituierte $C_{1-6}$-Alkyl-, Aryl-, Ar-$C_{1-4}$-alkyl-, $C_{1-6}$-Alkoxy-, Ar-$C_{1-4}$-alkoxy-, Aryloxy-, Carbamoyloxy-, $C_{1-6}$-Alkylthio-, $C_{2-6}$-Alkenyl-, $C_{2-6}$-Alkenyloxy-, Ar-$C_{1-4}$-alkylthio-, Ar-$C_{1-6}$-alkylsulfonyl-, Arylsulfonyl-, $C_{1-6}$-Alkylsulfonylamino-, Arylsulfonylamino-oder heterocyclische Gruppe als Substituent von $R^1$ und die substituierte stickstoffhaltige heterocyclische Gruppe als $R^6$ mindestens einen Substituenten aufweisen, der aus der Gruppe ausgewählt ist, die aus Halogenatomen, geschützten oder ungeschützten Hydroxylgruppen, geschützten oder ungeschützten Aminogruppen, geschützten oder ungeschützten Carboxylgruppen, unsubstituierten $C_{1-6}$-Alkylgruppen, $C_{1-6}$-Alkylgruppen, die durch eine geschützte oder ungeschützte Hydroxylgruppe substituiert sind, unsubstituierten oder Halogen-substituierten Arylgruppen, unsubstituierten oder Halogen-substituierten Aroylgruppen, unsubstituierten $C_{1-6}$-Alkoxygruppen, $C_{1-6}$-Alkoxygruppen, die durch eine $C_{1-6}$-Alkoxygruppe substituiert sind, $C_{1-6}$-Acylgruppen, Ar-$C_{1-4}$-alkylgruppen, Ar-$C_{2-6}$-alkenylgruppen, heterocyclischen Gruppen, Heterozyklus-CO-Gruppen, Oxogruppen, $C_{1-6}$-Alkylsulfonylgruppen und Arylsulfonylgruppen besteht; und die substituierte Aminogruppe als Substituent von $R^1$ mindestens jeweils einen Substituenten aufweist, der aus der Gruppe ausgewählt ist, die aus geschützten oder ungeschützten Hydroxylgruppen, unsubstituierten $C_{1-6}$-Alkylgruppen, $C_{1-6}$-Alkylgruppen, die durch eine geschützte oder ungeschützte Carboxyl- oder Hydroxylgruppe substituiert sind, cycloalkylgruppen, Arylgruppen, $C_{1-6}$-Acylgruppen, Ar-$C_{1-4}$-alkylgruppen, heterocyclischen Gruppen, unsubstituierten oder Oxo-substituierten Heterozyklus-CO-Gruppen, der Adamantylgruppe, $C_{1-6}$-Alkylsulfonylgruppen und Arylsulfonylgruppen besteht; wobei alle obigen heterocyclischen Gruppen aus der Gruppe ausgewählt sind, die aus den in der Definition von $R^6$ erwähnten stickstoffhaltigen heterocyclischen Gruppen und Furyl-, Thienyl-, Benzothienyl-, Pyranyl-, Isobenzofuranyl-, Oxazolyl-, Benzofuranyl-, Indolyl-, Benzimidazolyl-, Benzoxazolyl-, Benzothiazolyl-, Chinoxalyl-, Dihydrochinoxalyl-, 2,3-Dihydrobenzothienyl-, 2,3-Dihydrobenzopyrrolyl-, 2,3-Dihydro-4H-1-thianaphthyl-, 2,3-Dihydrobenzofuranyl-, Benzo[b]-dioxanyl-, Imidazo[2,3-a]pyridyl-, Benzo[b]piperazinyl-, Chromenyl-, Isothiazolyl-, Isoxazolyl-, Oxadiazolyl-, Pyridazinyl-, Isoindolyl- und Isochinolylgruppen besteht.

**2.** 1,2-Ethandiol-Derivat oder Salz desselben nach Anspruch 1, in dem $R^2$ ein Wasserstoffatom oder eine Hydroxyl-Schutzgruppe darstellt; die $nR^4$'s gleich oder verschieden voneinander sind und Wasserstoffatome oder $C_{1-6}$-Alkylgruppen darstellen; die $nR^5$'s Wasserstoffatome darstellen; und $R^6$ eine substituierte oder unsubstituierte stickstoffhaltige heterocyclische Gruppe darstellt, wobei die stickstoffhaltige heterocyclische Gruppe durch ein Kohlenstoffatom, das den heterocyclischen Ring bildet, am Kohlenstoffatom angebracht ist und aus der Gruppe ausgewählt ist, die aus Pyrrolyl-, Piperazinyl-, Imidazolyl-, Pyridyl-, Pyrimidinyl-, Chinolyl-, Chinolizinyl-, Tetrahydrochinolinyl-, Chinuclidinyl-, Thiazolyl- und Thiadiazolylgruppen besteht.

**3.** 1-(4-Benzyloxyphenyl)-2-[(1-methyl-5-imidazolyl)methoxy]ethanol oder ein Salz desselben.

**4.** Verwendung eines 1,2-Ethandiol-Derivats, das durch die folgende allgemeine Formel dargestellt wird, oder eines Salzes desselben für die Herstellung eines Arzneimittels zur Behandlung von zerebrovaskulärer Demenz, seniler Demenz, Alzheimer-Demenz, Folgen von ischämischer Enzephalopathie oder zerbraler Apoplexie bei einem Patienten:

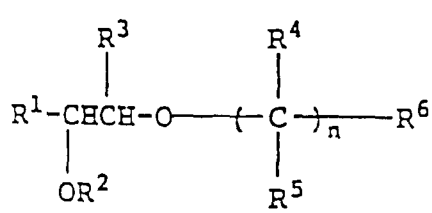

in der $R^1$ eine substituierte oder unsubstituierte Phenylgruppe darstellt; $R^2$ ein Wasserstoffatom, eine $C_{1-6}$-Alkylgruppe oder eine Hydroxyl-Schutzgruppe darstellt; $R^3$ ein Wasserstoffatom oder eine $C_{1-6}$-Alkylgruppe darstellt; die $nR^4$'s und $nR^5$'s gleich oder verschieden voneinander sind und Wasserstoffatome oder $C_{1-6}$-Alkylgruppen darstellen; $R^6$ eine substituierte oder unsubstituierte stickstoffhaltige heterocyclische Gruppe darstellt, wobei, wenn n null ist, die stickstoffhaltige heterocyclische Gruppe durch ein Kohlenstoffatom, das den heterocyclischen Ring bildet, am Sauerstoffatom oder, wenn n eine ganze Zahl von 1 bis 6 ist, am Kohlenstoffatom angebracht ist und aus der Gruppe ausgewählt ist, die aus Pyrrolyl-, Pyrrolidinyl-, Piperidyl-, Piperazinyl-, Imidazolyl-, Pyridyl-, Tetrahydropyridyl-, Pyrimidinyl-, Morpholinyl-, Thiomorpholinyl-, Chinolyl-, Chinolizinyl-, Tetrahydrochinolinyl-, Tetrahydroisochinolinyl-, Chinuclidinyl-, Thiazolyl-, Tetrazolyl-, Thiadiazolyl-, Pyrrolinyl-, Imidazolinyl-, Imidazolidinyl-, Pyrazolinyl-, Pyrazolidinyl-, Purinyl- und Indazolylgruppen besteht; und n 0 oder eine ganze Zahl von 1 bis 6 darstellt,

worin der Substituent an $R^1$ aus der Gruppe ausgewählt ist, die aus Halogenatomen, substituierten oder unsubstituierten Amino-, $C_{1-6}$-Alkyl-, Aryl-, Ar-$C_{1-4}$-alkyl-, $C_{1-6}$-Alkoxy-, Ar-$C_{1-4}$-alkoxy-, Aryloxy-, Carbamoyloxy-, $C_{1-6}$-Alkylthio-, $C_{2-6}$-Alkenyl-, $C_{2-6}$-Alkenyloxy-, Ar-$C_{1-4}$-alkylthio-, Ar-$C_{1-6}$-alkylsulfonyl-, Arylsulfonyl-, $C_{1-6}$-Alkylsulfonylamino-, Arylsulfonylamino-und heterocyclischen Gruppen, geschützten Aminogruppen, geschützten oder ungeschützten Hydroxylgruppen, der Nitrogruppe, der Oxogruppe und $C_{1-4}$-Alkylendioxygruppen besteht; die substituierte $C_{1-6}$-Alkyl-, Aryl-, Ar-$C_{1-4}$-alkyl-, $C_{1-6}$-Alkoxy-, Ar-$C_{1-4}$-alkoxy-, Aryloxy-, carbamoyloxy-, $C_{1-6}$-Alkylthio-, $C_{2-6}$-Alkenyl-, $C_{2-6}$-Alkenyloxy-, Ar-$C_{1-4}$-alkylthio-, Ar-$C_{1-6}$-alkylsulfonyl-, Arylsulfonyl-, $C_{1-6}$-Alkylsulfonylamino-, Arylsulfonylamino-oder heterocyclische Gruppe als der Substituent von $R^1$ und die substituierte stickstoffhaltige heterocyclische Gruppe als $R^6$ jeweils mindestens einen Substituenten aufweisen, der aus der Gruppe ausgewählt ist, die aus Halogenatomen, geschützten oder ungeschützten Hydroxylgruppen, geschützten oder ungeschützten Aminogruppen, geschützten oder ungeschützten Carboxylgruppen, unsubstituierten $C_{1-6}$-Alkylgruppen, $C_{1-6}$-Alkylgruppen, die durch eine geschützte oder ungeschützte Hydroxylgruppe substituiert sind, unsubstituierten oder Halogen-substituierten Arylgruppen, unsubstituierten oder Halogen-substituierten Aroylgruppen, unsubstituierten $C_{1-6}$-Alkoxygruppen, $C_{1-6}$-Alkoxygruppen, die durch eine $C_{1-6}$-Alkoxygruppe substituiert sind, $C_{1-6}$-Acylgruppen, Ar-$C_{1-4}$-alkylgruppen, Ar-$C_{2-6}$-alkenylgruppen, heterocyclischen Gruppen, Heterozyklus-CO-Gruppen, der Oxogruppe, $C_{1-6}$-Alkylsulfonylgruppen und Arylsulfonylgruppen besteht; und die substituierte Aminogruppe als der Substituent von $R^1$ jeweils mindestens einen Substituenten aufweist, der aus der Gruppe ausgewählt ist, die aus geschützten oder ungeschützten Hydroxylgruppen, unsubstituierten $C_{1-6}$-Alkylgruppen, $C_{1-6}$-Alkylgruppen, die durch eine geschützte oder ungeschützte Carboxyl- oder Hydroxylgruppe substituiert sind, Cycloalkylgruppen, Arylgruppen, $C_{1-6}$-Acylgruppen, Ar-$C_{1-4}$-alkylgruppen, heterocyclischen Gruppen, unsubstituierten oder Oxo-substituierten Heterozyklus-CO-Gruppen, der Adamantylqruppe, $C_{1-6}$-Alkylsulfonylqruppen und Arylsulfonylqruppen besteht; wobei alle obigen heterocyclischen Gruppen aus der Gruppe ausgewählt sind, die aus den in der Definition von $R^6$ erwähnten stickstoffhaltigen heterocyclischen Gruppen und Furyl-, Thienyl-, Benzothienyl-, Pyranyl-, Isobenzofuranyl-, Oxazolyl-, Benzofuranyl-, Indolyl-, Benzimidazolyl-, Benzoxazolyl-, Benzothiazolyl-, Chinoxalyl-, Dihydrochinoxalyl-, 2,3-Dihydrobenzothienyl-, 2,3-Dihydrobenzopyrrolyl-, 2,3-Dihydro-4H-1-thianaphthyl-, 2,3-Dihydrobenzofuranyl-, Benzo[b]dioxanyl-, Imidazo[2,3-a]pyridyl-, Benzo[b]piperazinyl-, Chromenyl-, Isothiazolyl-, Isoxazolyl-, Oxadiazolyl-, Pyridazinyl-, Isoindolyl- und Isochinolylgruppen besteht.

5. Verwendung eines 1,2-Ethandiol-Derivats oder eines Salzes desselben nach Anspruch 4, in dem $R^2$ ein Wasserstoffatom oder eine Hydroxyl-Schutzgruppe darstellt; die $nR^{4}$'s gleich oder verschieden voneinander sind und Wasserstoffatome oder $C_{1-6}$-Alkylgruppen darstellen; die $nR^{5}$'s Wasserstoffatome darstellen; und $R^6$ eine substituierte oder unsubstituierte stickstoffhaltige heterocyclische Gruppe darstellt, wobei, wenn n null ist, die stickstoffhaltige heterocyclische Gruppe durch ein Kohlenstoffatom, das den heterocyclischen Ring bildet, am Sauerstoffatom oder, wenn n eine ganze Zahl von 1 bis 6 ist, am Kohlenstoffatom angebracht ist und aus der Gruppe ausgewählt ist, die aus Pyrrolyl-, Pyrrolidinyl-, Piperidinyl-, Piperazinyl-, Imidazolyl-, Pyridyl-, Pyrimidinyl-, Morpholinyl-, Chinolyl-, Chinolizinyl-, Tetrahydrochinolinyl-, Chinuclidinyl-, Thiazolyl- und Thiadiazolylgruppen besteht.

6. Verwendung eines 1,2-Ethandiol-Derivats oder eines Salzes desselben nach Anspruch 4, in dem $R^6$ eine substituierte oder unsubstituierte stickstoffhaltige heterocyclische Gruppe darstellt, wobei, wenn n null ist, die stickstoffhaltige heterocyclische Gruppe durch ein Kohlenstoffatom, das den heterocyclischen Ring bildet, am Sauerstoffatom oder, wenn n eine ganze Zahl von 1 bis 6 ist, am Kohlenstoffatom angebracht ist und aus der Gruppe ausgewählt ist, die aus Pyrrolinyl, Piperidyl, Piperazinyl, Imidazolyl, Pyridyl, Morpholinyl, Thiomorpholinyl, Tetrahydropyridyl, Chinuclidinyl und Tetrazolyl besteht, der Substituent an $R^1$ aus der Gruppe ausgewählt ist, die aus Halogenatomen; $C_{1-6}$-Alkylgruppen; $C_{2-6}$-Alkenylgruppen; Phenylgruppen, die gegebenenfalls durch ein Halogenatom oder eine $C_{1-6}$-Alkyl- oder $C_{1-6}$-Alkoxygruppe substituiert sein können; Phenyl-$C_{1-4}$-alkylgruppen; $C_{1-6}$-Alkoxygruppen, die gegebenenfalls durch eine Pyridyl-, Furyl- oder Thienylgruppe substituiert sein können; Phenyl-$C_{1-4}$-alkoxygruppen, die gegebenenfalls durch ein Halogenatom oder eine $C_{1-6}$-Alkyl-, Hydroxyl- oder $C_{1-6}$-Alkoxygruppe substituiert sein können; einer Phenoxygruppe; Phenylaminocarbonyloxygruppen, die gegebenenfalls durch ein Halogenatom substituiert sein können; $C_{1-6}$-Alkylaminocarbonyloxygruppen; $C_{1-6}$-Alkylthiogruppen; einer Phenylsulfonylaminogruppe; Aminogruppen, die gegebenenfalls durch eine $C_{1-6}$-Alkylgruppe substituiert sein können; einer Hydroxylgruppe; einer Nitrogruppe; einer Oxogruppe; Phenyl-$C_{1-4}$-alkylthiogruppen; Phenyl-$C_{1-4}$-alkylsulfonylgruppen; einer Thienylgruppe; einer Pyridylgruppe; und $C_{1-4}$-Alkylendioxygruppen besteht; und der Substituent an $R^6$ aus der Gruppe ausgewählt ist, die aus Halogenatomen; einer Hydroxylgruppe; $C_{1-6}$-Alkylqruppen, die gegebenenfalls durch eine Hydroxylgruppe substituiert sein können; einer Aminogruppe; $C_{1-6}$-Acylgruppen, die gegebenenfalls durch eine Pyrrolidinylgruppe substituiert sein können; Phenyl-$C_{1-4}$-alkylgruppen; Phenyl-$C_{2-4}$-alkenylgruppen; Aroylgruppen, die durch ein Halogenatom substituiert sind; einer Pyridylgruppe und einer Oxogruppe besteht.

7. Verwendung eines 1,2-Ethandiol-Derivats oder eines Salzes desselben nach Anspruch 6, in dem $R^1$ eine Phenylgruppe, die gegebenenfalls durch ein Halogenatom substituiert sein kann; eine Phenyl-$C_{1-4}$-alkoxygruppe; eine Phenylgruppe; eine Thienylgruppe; eine Phenoxygruppe, die gegebenenfalls durch ein Halogenatom oder eine $C_{1-6}$-Alkoxygruppe substituiert sein kann; oder eine $C_{1-6}$-Alkoxygruppe darstellt, die gegebenenfalls durch eine Thienylgruppe substituiert sein kann; $R^2$ ein Wasserstoffatom darstellt; $R^3$ ein Wasserstoffatom darstellt; die $nR^4$'s und $nR^5$'s Wasserstoffatome darstellen; $R^6$ eine Morpholinylgruppe, eine Pyridylgruppe oder ein Imidazolylgruppe darstellt, die gegebenenfalls durch eine $C_{1-6}$-Alkylgruppe substituiert sein kann.

8. Verwendung eines 1,2-Ethandiol-Derivats oder eines Salzes desselben nach Anspruch 4, in dem das Derivat oder das Salz 1-(4-Benzyloxyphenyl)-2-[(1-methyl-5-imidazolyl)methoxy]ethanol oder ein Salz desselben ist.

9. Ein die Zerebralfunktion verbesserndes Mittel, umfassend ein 1,2-Ethandiol-Derivat, das durch die folgende allgemeine Formel dargestellt wird, oder ein Salz desselben:

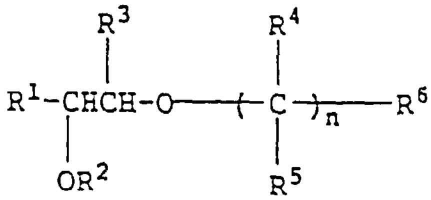

in der $R^1$, $R^2$, $R^3$, $R^4$, $R^5$, $R^6$ und n wie in Anspruch 4 definiert sind.

## Revendications

1. Dérivé de 1,2-éthanediol représenté par la formule générale suivante ou sel de celui-ci:

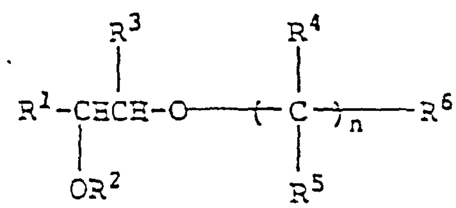

formule dans laquelle $R^1$ représente un groupe phényle substitué ou non substitué; $R^2$ représente un atome d'hydrogène, un groupe alcoyle en $C_{1-6}$ ou un groupe protecteur d'hydroxyle; $R^3$ représente un atome d'hydrogène ou un groupe alcoyle en $C_{1-6}$; les $nR^4$ et les $nR^5$ sont identiques ou différents l'un de l'autre et représentent des atomes d'hydrogène ou des groupes alcoyles en $C_{1-6}$; n représente un nombre entier de 1 à 6; et $R^6$ représente un groupe hétérocyclique contenant de l'azote, substitué ou non substitué, le groupe hétérocyclique contenant de l'azote étant lié à un atome de carbone par un atome de carbone de l'hétérocycle et étant choisi, lorsque n représente 1, dans le groupe consistant en les groupes pyrrolyle, pyrrolidinyle, pipérazinyle, imidazolyle, pyridyle, tétrahydropyridyle, pyrimidinyle, morpholinyle, thiomorpholinyle, quinoléyle, quinolizinyle, tétrahydroquinolinyle, tétrahydroisoquinolinyle, quinuclidinyle, thiazolyle, tétrazolyle, thiadiazolyle, pyrrolinyle, imidazolinyle, imidazolidinyle, pyrazolinyle, pyrazolidinyle, purinyle et indazolyle, ou étant choisi, lorsque n représente un nombre entier de 2 à 6, dans le groupe consistant en les groupes pyrrolyle, pyrrolidinyle, pipéridyle, pipérazinyle, imidazolyle, pyridyle, tétrahydropyridyle, pyrimidinyle, morpholinyle, thiomorpholinyle, quinoléyle, quinolizinyle, tétrahydroquinolinyle, tétrahydroisoquinolinyle, quinuclidinyle, thiazolyle, tétrazolyle, thiadiazolyle, pyrrolinyle, imidazolinyle, imidazolidinyle, pyrazolinyle, pyrazolidinyle, purinyle et indazolyle, formule dans laquelle le substituant de $R^1$ est choisi dans le groupes consistant en les atomes d'halogène, les groupes aminés substitués ou non substitués, les groupes alcoyle en $C_{1-6}$, aryle, aryl-$C_{1-4}$-alcoyle, alcoxy en $C_{1-6}$, aryl-$C_{1-4}$-alcoxy, aryloxy, carbamoyloxy, alcoylthio en $C_{1-6}$, alcényle en $C_{2-6}$, alcényloxy en $C_{2-6}$, aryl-$C_{1-4}$-alcoylthio, aryl-$C_{1-6}$-alcoylsulfonyle, arylsulfonyle, alcoylsulfonylamino en $C_{1-6}$, arylsulfonylamino et les groupes hétérocycliques, les groupes aminés protégés, les groupes

hydroxyles protégés ou non protégés, le groupe nitro, le groupe oxo et les groupes alcoylènedioxy en $C_{1-4}$; les groupes substitués alcoyle en $C_{1-6}$, aryle, aryl-$C_{1-4}$-alcoyle, alcoxy en $C_{1-6}$, aryl-$C_{1-4}$-alcoxy, aryloxy, carbamoyloxy, alcoylthio en $C_{1-6}$, alcényle en $C_{2-6}$, alcényloxy en $C_{2-6}$, aryl-$C_{1-4}$-alcoylthio, aryl-$C_{1-6}$-alcoylsulfonyle, arylsulfonyle, alcoylsulfonylamino en $C_{1-6}$, arylsulfonylamino ou le groupe hétérocyclique, en tant que substituant de $R^1$, et le groupe hétérocyclique substitué contenant de l'azote en tant que $R^6$ comportent chacun au moins un substituant choisi dans le groupe consistant en les atomes d'halogène, les groupes hydroxyles protégés ou non protégés, les groupes aminés protégés ou non protégés, les groupes carboxyliques protégés ou non protégés, les groupes alcoyles en $C_{1-6}$ non substitués, les groupes alcoyles en $C_{1-6}$ substitués par un groupe hydroxyle protégé ou non protégé, les groupes aryles non substitués ou substitués par un halogène, les groupes aroyles non substitués ou substitués par un halogène, les groupes alcoxy en $C_{1-6}$ non substitués, les groupes alcoxy en $C_{1-6}$ substitués par un groupe alcoxy en $C_{1-6}$, les groupes acyles en $C_{1-6}$, les groupes aryl-$C_{1-4}$-alcoyles, les groupes aryl-$C_{2-6}$-alcényles, les groupes hétérocycliques, les groupes hétérocycle-CO-, le groupe oxo, les groupes alcoylsulfonyles en $C_{1-6}$ et les groupes arylesulfonyles; et le groupe aminé substitué, en tant que substituant de $R^1$, comporte chaque fois au moins un substituant choisi dans le groupe consistant en les groupes hydroxyles protégés ou non protégés, les groupes alcoyles en $C_{1-6}$ non substitués, les groupes alcoyles en $C_{1-6}$ substitués par un groupe carboxylique ou hydroxyle protégé ou non protégé, les groupes cycloalcoyles, les groupes aryles, les groupes acyles en $C_{1-6}$, les groupes aryl-$C_{1-4}$-alcoyles, les groupes hétérocycliques, les groupes hétérocycle-CO- non substitués ou substitués par un oxo, le groupe adamantyle, les groupes alcoylsulfonyles en $C_{1-6}$ et les groupes arylesulfonyles; tous les groupes hétérocycliques ci-dessus étant choisis dans le groupe consistant en les groupes hétérocycliques contenant de l'azote mentionnés dans la définition de $R^6$ et les groupes furyle, thiényle, benzothiényle, pyranyle, isobenzofuranyle, oxazolyle, benzofuranyle, indolyle, benzimidazolyle, benzoxazolyle, benzothiazolyle, quinoxalyle, dihydroquinoxalyle, 2,3-dihydrobenzothiényle, 2,3-dihydrobenzopyrrolyle, 2,3-dihydro-4H-1-thianaphtyle, 2,3-dihydrobenzofuranyle, benzo[b]dioxanyle, imidazo[2,3-a]pyridyle, benzo[b]pipérazinyle, chroményle, isothiazolyle, isoxazolyle, oxadiazolyle, pyridazinyle, isoindolyle et isoquinoléyle.

**2.** Dérivé de 1,2-éthanediol ou sel de celui-ci selon la revendication 1, dans lequel $R^2$ représente un atome d'hydrogène ou un groupe protecteur d'hydroxyle; les $nR^4$ sont identiques ou différents l'un de l'autre et représentent des atomes d'hydrogène ou des groupes alcoyles en $C_{1-6}$; les $nR^5$ représentent des atomes d'hydrogène; et $R^6$ représente un groupe hétérocyclique contenant de l'azote, substitué ou non substitué, le groupe hétérocyclique contenant de l'azote étant lié à un atome de carbone par un atome de carbone formant l'hétérocycle et étant choisi dans le groupe consistant en les groupes pyrrolyle, pipérazinyle, imidazolyle, pyridyle, pyrimidinyle, quinoléyle, quinolizinyle, tétrahydroquinolinyle, quinuclidinyle, thiazolyle et thiadiazolyle.

**3.** Le 1-(4-benzyloxyphényl)-2-[(1-méthyl-5-imidazolyl)méthoxy]éthanol ou un sel de celui-ci.

**4.** Utilisation du dérivé de 1,2-éthanediol représenté par la formule générale suivante ou d'un sel de celui-ci pour préparer un médicament destiné au traitement de la démence cérébro-vasculaire, de la démence sénile, de la maladie d'Alzheimer, des suites de l'encéphalopathie ischémique ou de l'apoplexie cérébrale chez un malade:

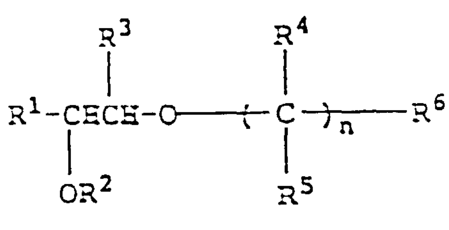

formule dans laquelle $R^1$ représente un groupe phényle substitué ou non substitué; $R^2$ représente un atome d'hydrogène, un groupe alcoyle en $C_{1-6}$ ou un groupe protecteur d'hydroxyle; $R^3$ représente un atome d'hydrogène ou un groupe alcoyle en $C_{1-6}$; les $nR^4$ et les $nR^5$ sont identiques ou différents l'un de l'autre et représentent des atomes d'hydrogène ou des groupes alcoyles en $C_{1-6}$; $R^6$ représente un groupe hétérocyclique contenant de l'azote, substitué ou non substitué, le groupe hétérocyclique contenant de l'azote étant lié à l'atome d'oxygène lorque n est zéro ou à un atome de carbone lorque n est un nombre entier de 1 à 6, ledit atome de carbone faisant partie de l'hétérocycle, et étant choisi par le groupe consistant en les groupes pyrrolyle, pyrrolidinyle, pipéridyle, pipérazinyle, imidazolyle, pyridyle, tétrahydropyridyle, pyrimidinyle, morpholinyle, thiomorpholinyle, quinoléyle, quinolizinyle, tétrahydroquinolinyle, tétrahydroisoquinolinyle, quinuclidinyle, thiazolyle, tétrazolyle, thiadiazolyle, pyrrolinyle, imidazolinyle, imidazolidinyle, pyrazolinyle, pyrazolidinyle, purinyle et indazolyle; et n représente 0 ou un

nombre entier de 1 à 6, formule dans laquelle le substituant de $R^1$ est choisi dans le groupe consistant en les atomes d'halogène, les groupes aminés substitués ou non substitués, les groupes alcoyle en $C_{1-6}$, aryle, aryl-$C_{1-4}$-alcoyle, alcoxy en $C_{1-6}$, aryl-$C_{1-4}$-alcoxy, aryloxy, carbamoyloxy, alcoylthio en $C_{1-6}$, alcényle en $C_{2-6}$, alcényloxy en $C_{2-6}$, aryl-$C_{1-4}$-alcoylthio, aryl-$C_{1-6}$-alcoylsulfonyle, arylsulfonyle, alcoylsulfonylamino en $C_{1-6}$, arylsulfonylamino et les groupes hétérocycliques, les groupes aminés protégés, les groupes hydroxyles protégés ou non protégés, le groupe nitro, le groupe oxo et les groupes alcoylènedioxy en $C_{1-4}$; les groupes substitués alcoyle en $C_{1-6}$, aryle, aryl-$C_{1-4}$-alcoyle, alcoxy en $C_{1-6}$, aryl-$C_{1-4}$-alcoxy, aryloxy, carbamoyloxy, alcoylthio en $C_{1-6}$, alcényle en $C_{2-6}$, alcényloxy en $C_{2-6}$, aryl-$C_{1-4}$-alcoylthio, aryl-$C_{1-6}$-alcoylsulfonyle, arylsulfonyle, alcoylsulfonylamino en $C_{1-6}$, arylsulfonylamino ou le groupe hétérocycle, en tant que substituant de $R^1$, et le groupe hétérocyclique substitué contenant de l'azote, en tant que $R^6$, comportent chacun au moins un substituant choisi dans le groupe consistant en les atomes d'halogène, les groupes hydroxyles protégés ou non protégés, les groupes aminés protégés ou non protégés, les groupes carboxyliques protégés ou non protégés, les groupes alcoyles en $C_{1-6}$ non substitués, les groupes alcoyles en $C_{1-6}$ substitués par un groupe hydroxyle protégé ou non protégé, les groupes aryles non substitués ou substitués par un halogène, les groupes aroyles non substitués ou substitués par un halogène, les groupes alcoxy en $C_{1-6}$ non substitués, les groupes alcoxy en $C_{1-6}$ substitués par un groupe alcoxy en $C_{1-6}$, les groupes acyles en $C_{1-6}$, les groupes aryl-$C_{1-4}$-alcoyles, les groupes aryl-$C_{2-6}$-alcényles, les groupes hétérocycliques, les groupes hétérocycle-CO-, le groupe oxo, les groupes alcoylsulfonyles en $C_{1-6}$ et les groupes arylesulfonyles; et le groupe aminé substitué, en tant que substituant de $R^1$, comporte chaque fois au moins un substituant choisi dans le groupe consistant en les groupes hydroxyles protégés ou non protégés, les groupes alcoyles en $C_{1-6}$ non substitués, les groupes alcoyles en $C_{1-6}$ substitués par un groupe carboxylique ou hydroxyle protégé ou non protégé, les groupes cycloalcoyles, les groupes aryles, les groupes acyles en $C_{1-6}$, les groupes aryl-$C_{1-4}$-alcoyaes, les groupes hétérocycliques, les groupes hétérocycle-CO- non substitués ou substitués par un oxo, le groupe adamantyle, les groupes alcoylsulfonyles en $C_{1-6}$ et les groupes arylesulfonyles; tous les groupes hétérocycliques ci-dessus étant choisis dans le groupe consistant en les groupes hétérocycliques contenant de l'azote mentionnés dans la définition de $R^6$ et les groupes furyle, thiényle, benzothiényle, pyranyle, isobenzofuranyle, oxazolyle, benzofuranyle, indolyle, benzimidazolyle, benzoxazolyle, benzothiazolyle, quinoxalyle, dihydroquinoxalyle, 2,3-dihydrobenzothiényle, 2,3-dihydrobenzopyrrolyle, 2,3-dihydro-4H-1-thianaphtyle, 2,3-dihydrobenzofuranyle, benzo[b]dioxanyle, imidazo[2,3-a]pyridyle, benzo[b]pipérazinyle, chroményle, isothiazolyle, isoxazolyle, oxadiazolyle, pyridazinyle, isoindolyle et isoquinoléyle.

**5.** Utilisation d'un dérivé de 1,2-éthanediol ou d'un sel de celui-ci selon la revendication 4, dans laquelle $R^2$ représente un atome d'hydrogène ou un groupe protecteur d'hydroxyle; les $nR^4$ sont identiques ou différents l'un de l'autre et représentent des atomes d'hydrogène ou des groupes alcoyles en $C_{1-6}$; les $nR^5$ représentent des atomes d'hydrogène; et $R^6$ représente un groupe hétérocyclique contenant de l'azote, substitué ou non substitué, le groupe hétérocyclique contenant de l'azote étant lié à l'atome d'oxygène lorsque n est zéro ou à un atome de carbone lorsque n est un nombre entier de 1 à 6, ledit atome de carbone faisant partie de l'hétérocycle, et étant choisi dans le groupe consistant en les groupes pyrrolyle, pyrrolidinyle, pipéridinyle, pipérazinyle, imidazolyle, pyridyle, pyrimidinyle, morpholinyle, quinoléyle, quinolizinyle, tétrahydroquinolinyle, quinuclidinyle, thiazolyle et thiadiazolyle.

**6.** Utilisation d'un dérivé de 1,2-éthanediol ou d'un sel de celui-ci selon la revendication 4, dans laquelle $R^6$ représente un groupe hétérocyclique contenant de l'azote, substitué ou non substitué, le groupe hétérocyclique contenant de l'azote étant lié à l'atome d'oxygène lorsque n est zéro ou à un atome de carbone lorsque n est un nombre entier de 1 à 6, ledit atome de carbone faisant partie de l'hétérocycle, et étant choisi dans le groupe consistant en les groupes pyrrolinyle, pipéridyle, pipérazinyle, imidazolyle, pyridyle, morpholinyle, thiomorpholinyle, tétrahydropyridyle, quinuclidinyle et tétrazolyle, le substituant de $R^1$ étant choisi dans le groupe consistant en les atomes d'halogène; les groupes alcoyles en $C_{1-6}$, les groupes alcényles en $C_{2-6}$; les groupes phényles qui peuvent être substitués cas échéant par un atome d'halogène ou un groupe alcoyle en $C_{1-6}$ ou alcoxy en $C_{1-6}$; les groupes phényl-$C_{1-4}$-alcoyles; les groupes alcoxy en $C_{1-6}$ qui peuvent être substitués cas échéant par un groupe pyridyle, furyle ou thiényle, les groupes phényl-$C_{1-4}$-alcoxy qui peuvent être substitués cas échéant par un atome d'halogène ou un groupe alcoyle en $C_{1-6}$, un groupe hydroxyle ou alcoxy en $C_{1-6}$; un groupe phénoxy; les groupes phénylaminocarbonyloxy qui peuvent être substitués cas échéant par un atome d'halogène; les groupes $C_{1-6}$-alcoylaminocarbonyloxy; les groupes alcoylthio en $C_{1-6}$; le groupe phénylsulfonylamino; les groupes aminés qui peuvent être substitués cas échéant par un groupe alcoyle en $C_{1-6}$, le groupe hydroxyle; le groupe nitro; le groupe oxo; les groupes phényl-$C_{1-4}$-alcoylthio; les groupes phényl-$C_{1-4}$-alcoylsulfonyles; le groupe thiényle; le groupe pyridyle; et les groupes alcoylènedioxy en $C_{1-4}$; et le substituant de $R^6$ est choisi dans le groupe consistant en les atomes d'halogène; le groupe hydroxyle; les groupes alcoyles en $C_{1-6}$ qui peuvent être substitués cas échéant par un groupe hydroxyle; le groupe aminé; les groupes acyles en $C_{1-6}$ qui peuvent être substitués cas échéant par un groupe pyrrolidinyle; les groupes phényl-$C_{1-4}$-alcoyles; les groupes phényl-$C_{2-4}$-alcényles; les groupes aroyles qui

peuvent être substitués par un atome d'halogène; le groupe pyridyle et le groupe oxo.

**7.** Utilisation d'un dérivé de 1,2-éthanediol ou d'un sel de celui-ci selon la revendication 6, dans laquelle $R^1$ représente un groupe phényle qui peut être substitué cas échéant par un atome d'halogène; un groupe phényle-$C_{1-4}$-alcoxy; un groupe phényle, un groupe thiényle, un groupe phénoxy qui peut être substitué cas échéant par un atome d'halogène ou un groupe alcoxy en $C_{1-6}$; ou un groupe alcoxy en $C_{1-6}$ qui peut être substitué cas échéant par un groupe thiényle; $R^2$ représente un atome d'hydrogène; $R^3$ représente un atome d'hydrogène; les $nR^4$ et les $nR^5$ représentent des atomes d'hydrogène; $R^6$ représente un groupe morpholinyle, un groupe pyridyle ou un groupe imidazolyle qui peut être substitué cas échéant par un groupe alcoyle en $C_{1-6}$.

**8.** Utilisation d'un dérivé de 1,2-éthanediol ou d'un sel de celui-ci selon la revendication 4, dans laquelle ledit dérivé ou sel est le 1-(4-benzyloxyphényl)-2-[(1-méthyl-5-imidazolyl)méthoxy]éthanol ou un sel de celui-ci.

**9.** Agent d'amélioration de la fonction cérébrale, qui comprend un dérivé de 1,2-éthanediol représenté par la formule générale suivante ou un sel de celui-ci:

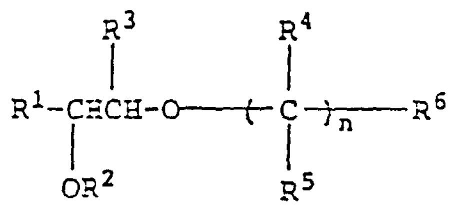

formule dans laquelle $R^1$, $R^2$, $R^3$, $R^4$, $R^5$, $R^6$ et n sont tels que défini à la revendication 4.